# EUROPEAN PATENT APPLICATION

(11) **EP 1 480 042 A2**
(43) Date of publication of application: **24.11.2004**
(21) Application number: 04020521.3
(22) Date of filing: 01.12.1999
(51) Int. Cl.: G01N 33/68

(54) **Method of screening compounds interacting with proteins of the p53 family**

(30) Priority: 02.12.1998 US 110542 P
(62) Divisional of application: 99956270.5
(71) Applicant: Pfizer Products Inc., Groton, Connecticut 06340 (US)
(72) Inventor: Coffey, Heather Anne, Groton, Connecticut 06340 (US); Connell, Richard Damian, Groton, Connecticut 06340 (US); Foster, Barbara Ann, Groton, Connecticut 06340 (US); Rastinejad, Farzan, Groton, Connecticut 06340 (US)
(74) Representative: Eddowes, Simon

(57) **Abstract**

There is provided a method for screening an organic non-peptide compound for interaction with one or more domains of either a wild-type human protein of the p53 family or a mutant human protein of the p53 family, which interaction can stabilize a functional conformation thereof under physiological conditions and promote wild-type activity of the protein of the p53 family, or restore wild-type activity to a mutant protein of the p53 family, which method comprises contacting said human protein of the p53 family, or one or more domains thereof, and said organic non-peptide compound, and monitoring the interaction thereof.

## Description

### I. Field of the Invention

The invention is in the field of cancer treatment. The present invention provides organic non-peptide compounds capable of interacting with a tumor suppressor protein of the p53 family and stabilizing a functional conformation therein. The invention is particularly applicable to stabilizing mutant forms of tumor suppressor proteins in patients where correcting the functional capacity of such proteins can facilitate treatment for cancer. Methods for screening for such compounds are also provided.

### II. Background of the Invention

The primary structure of a protein is the particular sequence of amino acid building blocks that are linked together to form the protein's polypeptide chain(s). These polypetide chains are, in turn, folded into a three-dimensional structure. A number of diverse diseases are now thought to arise from a conformational perturbation in the three-dimensional structure of a cellular protein (see for reviews Thomas *et al*., 1995, TIBS 20:456-459; Carrell *et al*., 1997, Lancet 350:134-138). For example, Alzheimer's disease is caused by misfolding and subsequent aggregation of beta-amyloid protein, leading to impairment of cell function. Similarly, the etiological agents for Creutzfeld-Jakob disease, prions, are thought to cause the disease by initiating a chain reaction converting normal prion proteins to misfolded prion proteins.

Proteins that adopt abnormal conformations may do so either because they are inherently susceptible to misfolding or because they have mutations that thermodynamically destabilize the mutant protein relative to wild-type protein. A prime example of missense mutations leading to disease is the tumor suppressor protein p53.

Wild-type p53 functions as a transcriptional regulator to coordinately control multiple pathways in cell cycling, apoptosis, and angiogenesis. The cellular pathways that monitor cellular stresses, such as DNA damage, mitotic spindle mis-assembly, and hypoxia, all appear to converge on p53. Loss of p53 activity can lead to uncontrolled proliferation of the affected cells and tumor growth. Although loss of p53 activity may or may not, by itself, be the trigger to transforming a cell into a cancerous cell, detectable cancers are more common and likely to grow in persons with p53 mutations. In fact, mutants of p53 are the most common genetic aberration in cancer.

Recently, two additional proteins, p73 and p63, have been identified with homology to p53 *(see* for review Kaelin, 1999, J. Natl. Cancer Inst. 91:594-598; *see also* Yang *et al*., 1998, Molecular Cell 2(3):305-16; and Yoshikawa *et al*., 1999, Oncogene 18(22):3415-21). p51 has also been termed p40, p51, KET or p73L. Not only do these proteins share amino acid sequence homology with p53, but they can also activate p53 responsive promoters and induce apoptosis. Furthermore, the genes encoding these proteins appear ancestrally related to p53. Thus, there is an art-recognized family of proteins related p53 that have similar functions and related amino acid sequences.

p53 is a complex macromolecule with three independent functional domains: an N-terminus that includes a transcriptional activation domain (approximately amino acids 1-43); a central portion that encodes a DNA binding domain (DBD) (approximately amino acids 100-300); and a C-terminal portion that serves as an oligomerization domain (approximately amino acids 319-360). The crystal structure of the p53 DBD shows a roughly spherical globular domain with high beta-sheet content.

p53 activity is highly dependent on the ability of the protein to maintain its functional conformation. Analysis of tumors derived from many different cancers reveals that the DBD is frequently mutated. Friedlander *et al*., 1996, J. Biol. Chem. 271:25468-25478. Although there are a large variety of point mutations that occur within the p53 DNA binding domain in major cancers (Pavletich *et al*., 1993, Genes & Development 7, 2556-2564), specific residue positions within the p53 DBD, known as hot-spots, are mutated with unusually high occurrences. Hot-spot mutations commonly found in human tumors are somewhat randomly dispersed throughout the DBD. When exposed to urea, the p53 DBDs of all frequently mutated forms of p53 are less stable than the wild-type DBD (Bullock *et al*., *supra)*. Additionally, p53 mutants often associate with heat shock proteins in cells, leading to speculation that they are less capable of retaining native conformation (Finlay *et al*., 1988, Molecular and Cellular Biology 8:531-39).

Interactions with the C-terminal domain of p53 have been found to activate the cell-cycle arrest properties of p53. Specifically, injection of C-terminal specific p53 antibody into cycling cells could arrest them (Mercer *et al*., 1982, Proc. Nat. Acad. Sci.:USA 79, 6309-6312). More detailed studies demonstrated that the C-terminal domain regulates the DNA binding activity of the DBD domain. For example, Hupp *et al*. found that the monoclonal antibody Pab 421, which interacts with residues 373-381 of the p53 C-terminal domain, is capable of enhancing DNA binding activity of certain mutant forms of p53 (Hupp *et al*., 1993 , Nucleic Acids Research 21: 3167-3174). Thus, Hupp and colleagues focused on antibodies and peptides that neutralize an independent negative regulatory domain in the C terminus in an attempt to restore p53 function (Selivanova *et al*., 1997, Nature Med. 3, 632-638). However, the position 273 mutants which are restored by this approach differ from other common mutants in that they retain a high basal DNA binding activity and display thermodynamic stability features similar to the wild-type protein (Bullock *et al*., 1997, Proc. Nat. Acad.. Sci.:USA 94, 14338-143421).

Other researchers in the field have argued that the development of a compound that binds the N-terminal domain of mutant p53 is the most effective route to rescuing wild-type p53 activity. For example, Friedlander *et al*. tested a number of different monoclonal antibodies that bound to defined epitopes on p53 for the ability to promote DNA binding activity of temperature sensitive p53 mutants. Friedlander *et al*., 1996, J. Biol. Chem. 271, 25468-25478. While the C terminal specific antibody PAb 421 did restore DNA binding function to mutant p53 at lower temperatures, N terminal specific p53 antibodies, and in particular monoclonal antibody Pab1801, were more effective at promoting DNA binding activity of temperature sensitive p53 mutants at elevated temperatures. Based on these findings Friedlander *et al*. speculated that the development of a small molecule that mimics the 1801 epitope recognition region by binding to the N terminus would facilitate wild-type DNA binding activity in mutant p53. Notably, Friedlander *et al*. demonstrated that an antibody specific to an epitope in the central portion (DBD domain) of the p53 protein had no effect on DNA binding activity. As one explanation of their results, Friedlander *et al*. hypothesized that the conformation of one domain within a protein was stabilized by using a distant domain. Bullock *et al*. demonstrated that the change in thermodynamic stability in commonly occurring p53 DNA binding domain mutants is rather small, and speculated that development of a small molecule therapy for p53 such as that suggested by Friedlander et *al*. (i.e., molecules that bind to the N terminus) could be feasible. Bullock *et al*., 1997, *supra*.

Other, more global, approaches to identifying anticancer compounds have focused on assaying the direct, anti-tumor activities of small molecules in cell-based (e.g., tumor cell lines) or animal assays. A number of small molecules with possible antitumor activity have been described. Mazerska *et al*., 1990, Anti-Cancer Drug Design 5, 169-187; Su *et al*., 1995, J. Med. Chem. 38, 3226-3235; Nagy *et al*., 1996, Anticancer Research 16, 1915-1918; Wuonola *et al*., 1997, Anticancer Research 17, 3409-23. Mazerska *et al*. describe a series of nitro-9-aminoacridines with a nitro group attached to the acridine group whose anti-tumor properties were attributed to their ability to bind DNA and produce covalent interstrand crosslinks. Su *et al*. describe a series of 9-Anilinoacridine derivatives with various positions of the anilino and acridine ring system substituted that were developed as topoisomerase II inhibitors. Nagy *et al*. describe a series of phenothiazine-related compounds attached via a short carbon linker to a urea or phthalimido based group. Nagy *et al*. postulated that the anti-tumor cell activity of this class of compounds derived from their ability to react with calcium channels and calmodulin. Wuonola *et al*., *supra*, describe phenothiazine compounds that are similar to the compounds described by Nagy *et al*., *supra*.

To date, a small organic non-peptide molecule that interacts with a protein of the p53 family to restore or stabilize wild-type activities, such as tumor suppression activity, has not been reported. Further, the discovery of such compounds has been precluded by the lack of a high through-put screen or assay.

### III. Summary of the Invention

Recognizing the importance of identifying compounds that can conformationally stabilize thermodynamically unstable proteins or misfolding proteins associated with human diseases, and cognizant of the lack of a high through-put assay system in which such compounds might be rapidly identified, the inventors have investigated the use of isolated mutant p53 DNA binding domain (DBD) in *in vitro* and *in vivo* assays as a model system in which to rapidly identify agents that conformationally stabilize mutant p53. The invention provides a quick, reliable and accurate method for objectively identifying compounds, including human pharmaceuticals, that promote wild-type activity in a protein of the p53 family.

Accordingly, the present invention provides the first demonstration that non-peptide organic compounds can interact with a protein of the p53 family and promote its wild-type activity. At or near physiological temperatures, these active compounds promoted a wild-type activity of p53 in not only a variety of mutant p53 proteins, but also wild-type p53 proteins. Such compounds have important use as anti-cancer pharmaceuticals. Thus, the invention provides a novel approach and compounds useful for antitumor therapy in cancers with mutant or wild-type activity of a protein of the p53 family.

In one aspect, the invention provides a method of promoting a wild-type activity in a mutant form of a human protein of the p53 family, wherein one or more functional activities of the protein are at least partially impaired by the inability of the protein to maintain a functional conformation under physiological conditions, the method comprising the steps of contacting the mutant protein with an organic non-peptide compound that is capable of binding to one or more domains in the mutant protein under physiological conditions and stabilizing a functional conformation therein, and permitting the stabilized protein to interact with one or more macromolecules that participate in the wild type activity. The human protein of the p53 family can be, for example, p53, p63 or p73. In preferred embodiments, the organic, non-peptide compound interacts with p53, and even more preferably, with the DNA binding domain of p53

The invention also provides, in another embodiment, a method of treating a human subject for a disease state associated with expression of a mutant protein of the p53 family that has one or more diminished wild-type activities, comprising the steps of administering to the subject an organic non-peptide compound that is capable of binding to one or more domains in the mutant protein under physiological conditions, and stabilizing a functional conformation therein; and permitting the stabilized protein in the patient to interact with one or more macromolecules that participate in the wild-type activity. In yet another embodiment, the invention provides a method of treating a human subject for cancer comprising the steps of: administering to the subject an organic non-peptide compound that is capable of binding to one or more domains of a human protein of the p53 family under physiological conditions, and stabilizing a functional conformation therein, and permitting the stabilized protein to interact with one or more macromolecules that participate in a wild-type activity of the protein.

In one aspect, organic non-peptide compounds for use in the invention can be a compound containing both a hydrophobic group (e.g., a planar polycyclic) and a cationic group (preferably an amine) joined together by a linker of a specific length.

In a preferred aspect, the organic non-peptide compounds for use in the invention are selected from the group consisting of: 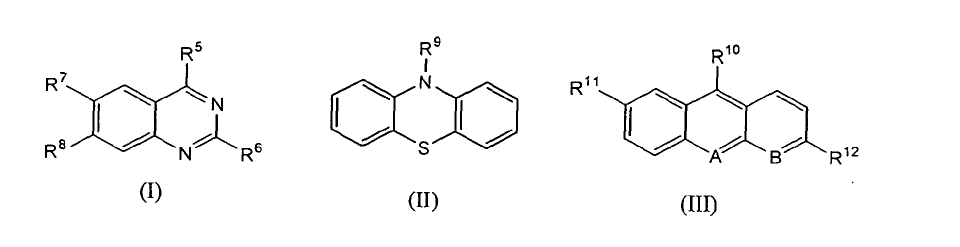 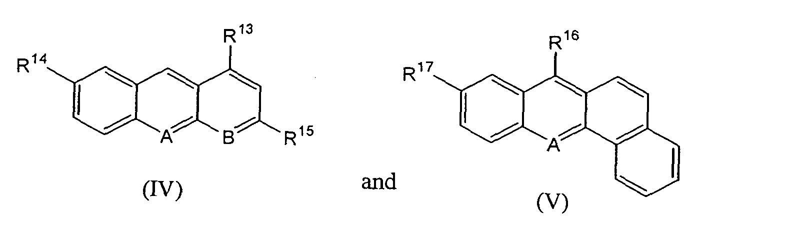 wherein, for group I, 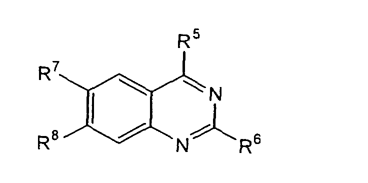 R⁵ is ―N―R¹⁸R¹⁹, where
R¹⁸ is H, (C₁-C₆)alkyl, or phenyl, and
R¹⁹ is H, (C₁-C₆)alkyl, (C₃-C₁₀)cycloalkyl, or phenyl, wherein said alkyl, cycloalkyl or phenyl group is optionally substituted with hydroxy, (C₃-C₈)cycloheteroalkyl,
―CON R¹⁸(CH₂)ₚNR²⁰R²¹, ―(CH₂)ₚ-(CHR²²)ₘ-(CH₂)ₙ―NR²⁰R²¹, or
―(CH₂)ₚ-(CHR²²)ₘ-(CH₂)n―NR²⁰R²¹, wherein p is 0-5, m is 0-5, n is 0-5, R²² is hydroxy or (C₁-C₆)alkyl, and
R²⁰ and R²¹ are each, independently selected from:
(a) H, (C₁-C₁₂)alkyl, (C₃-C₁₂)cycloalkyl, (C₃-C₁₀)heterocycloalkyl, (C₆-C₁₀)aryl, (C₅-C₉)heteroaryl, (C₁-C₆)alkyl(C₆-C₁₂)aryl, wherein said groups are optionally substituted by one or more hydroxy, halo, amino, trifluoromethyl, (C₁-C₆)alkyl, (C₁-C₆)alkoxy, (C₁-C₆)alkyl(C₃-C₁₀)heterocycloalkyl, or (C₁-C₆)alkyl(C₆-C₁₀)aryl; or
(b) NR²⁰R²¹ taken together represent hydrogen, morpholine, or 4-(C₁-C₆) alkylpiperizine; R⁶ is

(a) (C₁-C₆)alkyl or (C₂-C₈)alkenyl, each optionally substituted by one or more phenyl groups, or
(b) phenyl substituted by halo, (C₁-C₆)alkoxy; and
   R⁷ and R⁸ are the same, or different, and are selected from H, nitro, (C₁-C₆)alkoxy, or halogen selected from fluoro, chloro, and bromo;
wherein, for group II, 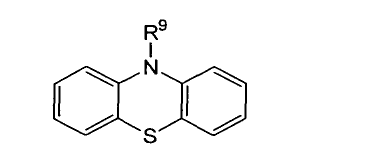 R⁹ is (C₁-C₆)alkyl, (C₃-C₁₀)cycloalkyl, or phenyl, wherein said alkyl, cycloalkyl or phenyl group is optionally substituted with hydroxy, (C₃-C₈)cycloheteroalkyl, ―CON R¹⁸(CH₂)ₚNR²⁰R²¹, ―(CH₂)ₚ-(CHR²²)ₘ-(CH₂)ₙ―NR²⁰R²¹, or
―(CH₂)ₚ-(CHR²²)ₘ-(CH₂)ₙ-NR²⁰R²¹, wherein p is 0-5, m is 0-5, n is 0-5, R²² is hydroxy or (C₁-C₆)alkyl, and
R²⁰ and R²¹ are each independently selected from H, (C₁-C₁₂)alkyl, (C₃-C₁₂)cycloalkyl, (C₃-C₁₀)heterocycloalkyl, (C₆-C₁₀)aryl, (C₅-C₉)heteroaryl, (C₁-C₆)alkyl(C₆-C₁₂)aryl, wherein said groups are optionally substituted by one or more hydroxy, halo, amino, trifluoromethyl, (C₁-C₆)alkyl, (C₁-C₆)alkoxy, (C₁-C₆)alkyl(C₃-C₁₀)heterocycloalkyl, (C₁-C₆)alkyl(C₅-C₉)heteroaryl, or (C₁-C₆)alkyl(C₆-C₁₀)aryl;
wherein, for group III, 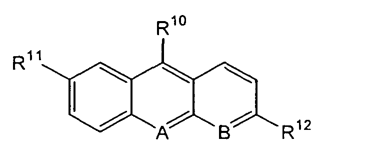 R¹⁰ is ―N―R¹⁸R¹⁹, where
R¹⁸ is H, (C₁-C₆)alkyl, or phenyl, and
R¹⁹ is H, (C₁-C₆)alkyl, (C₃-C₁₀)cycloalkyl, or phenyl, wherein said alkyl, cycloalkyl or phenyl group is optionally substituted with hydroxy, (C₃-C₈)cycloheteroalkyl,
―CON R¹⁸(CH₂)ₚNR²⁰R²¹, ―(CH₂)ₚ-(CHR²²)ₘ-(CH₂)ₙ―NR²⁰R²¹, or
―(CH₂)ₚ-(CHR²²)ₘ-(CH²)ₙ―NR²⁰R²¹, wherein p is 0-5, m is 0-5, n is 0-5, R²² is hydroxy or (C₁-C₆)alkyl, and
R²⁰ and R²¹ are each, independently selected from:
(a) H, (C₁-C₁₂)alkyl, (C₃-C₁₂)cycloalkyl, (C₃-C₁₀)heterocycloalkyl, (C₆-C₁₀)aryl, (C₅-C₉)heteroaryl, (C₁-C₆)alkyl(C₆-C₁₂)aryl, wherein said groups are optionally substituted by one or more hydroxy, halo, amino, trifluoromethyl, (C₁-C₆)alkyl, (C₁-C₆)alkoxy, (C₁-C₆)alkyl(C₃-C₁₀)heterocycloalkyl, (C₁-C₆)alkyl(C₅-C₉)heteroaryl, or (C₁-C₆)alkyl(C₆-C₁₀)aryl; or
(b) NR²⁰R²¹ taken together represent hydrogen, morpholine, or 4-(C₁-C₆) alkylpiperizine;
A and B are the same or different, and each represents carbon or nitrogen; and
R¹¹ and R¹² are the same, or different, and are selected from H, nitro, (C₁-C₆)alkoxy, or halogen selected from fluoro, chloro, and bromo;
wherein, for group IV, 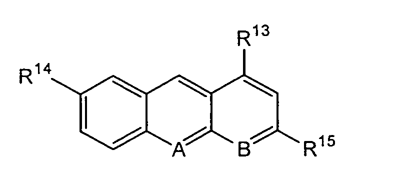 R¹³ is ―N―R¹⁸R¹⁹, where
R¹⁸ is H, (C₁-C₆)alkyl, or phenyl, and
R¹⁹ is H, (C₁-C₆)alkyl, (C₃-C₁₀)cycloalkyl, or phenyl, wherein said alkyl, cycloalkyl or phenyl group is optionally substituted with hydroxy, (C₃-C₈)cycloheteroalkyl,
―CON R¹⁸(CH₂)ₚNR²⁰R²¹, ―(CH₂)ₚ-(CHR²²)ₘ-(CH₂)ₙ―NR²⁰R²¹, or
―(CH₂)ₚ-(CHR²²)ₘ-(CH₂)ₙ―NR²⁰R²¹, wherein p is 0-5, m is 0-5, n is 0-5, R²² is hydroxy or (C₁-C₆)alkyl, and
R²⁰ and R²¹ are each, independently selected from:
(a) H, (C₁-C₁₂)alkyl, (C₃-C₁₂)cycloalkyl, (C₃-C₁₀)heterocycloalkyl, (C₁-C₆)alkyl(C₅-C₉)heteroaryl, (C₅-C₉)heteroaryl, (C₆-C₁₀)aryl, and (C₁-C₆)alkyl(C₆-C₁₀)aryl, wherein said groups are optionally substituted by one or more hydroxy, halo, amino, trifluoromethyl, (C₁-C₆)alkyl, (C₁-C₆)alkoxy, (C₁-C₆)alkyl(C₃-C₁₀)heterocycloalkyl, (C₁-C₆)alkyl(C₅-C₉)heteroaryl and (C₁-C₆)alkyl(C₆-C₁₀)aryl; or
(b) NR²⁰R²¹ taken together represent hydrogen, morpholine, or 4-(C₁-C₆) alkylpiperizine;
A and B are the same or different, and each represents carbon or nitrogen; and
R¹⁴ and R¹⁵ are the same, or different, and are selected from H, nitro, (C₁-C₆)alkoxy, or halogen selected from fluoro, chloro, and bromo; and
wherein, for group V, 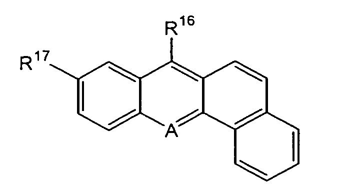 A is carbon or nitrogen;
R¹⁶ is ―N―R¹⁸R¹⁹, where
R¹⁸ is H, (C₁-C₆)alkyl, or phenyl, and
R¹⁹ is H, (C₁-C₆)alkyl, (C₃-C₁₀)cycloalkyl, or phenyl, wherein said alkyl, cycloalkyl or phenyl group is optionally substituted with hydroxy, (C₃-C₈)cycloheteroalkyl,
―CON R¹⁸(CH₂)ₚNR²⁰R²¹, ―(CH₂)ₚ-(CHR²²)ₘ-(CH₂)ₙ-NR²⁰R²¹, or ―(CH₂)ₚ(CHR²²)ₘ-(CH₂)ₙ―NR²⁰R²¹, wherein p is 0-5, m is 0-5, n is 0-5, R²² is hydroxy or (C₁-C₆)alkyl, and
R²⁰ and R²¹ are each, independently selected from:
(a) H, (C₁-C₁₂)alkyl, (C₃-C₁₂)cycloalkyl, (C₃-C₁₀)heterocycloalkyl, (C₆-C₁₀)aryl, (C₅-C₉)heteroaryl, (C₁-C₆)alkyl(C₆-C₁₀)aryl, and (C₁-C₆)alkyl(C₅-C₉)heteroaryl, or wherein said groups are optionally substituted by one or more hydroxy, halo, amino, trifluoromethyl, (C₁-C₆)alkyl, (C₁-C₆)alkoxy, (C₁-C₆)alkyl(C₃-C₁₀)heterocycloalkyl, (C₁-C₆)alkyl(C₅-C₉)heteroaryl, or (C₁-C₆)alkyl(C₆-C₁₀)aryl; or
(b) NR²⁰R²¹ taken together represent hydrogen, morpholine, or 4-(C₁-C₆) alkylpiperizine; and
R¹⁷ selected from H, nitro, (C₁-C₆)alkoxy, or halogen selected from fluoro, chloro, and bromo.

Additionally, many of the compounds useful in the practice of the invention are themselves novel, and the description hewrein of such compounds defines a further aspect of the invention.

The invention also provides, in another aspect, a method of designing additional compounds that promote a wild-type activity of a protein of the p53 family. The method entails using one of the active compounds of the invention to generate a hypothesis, identifying a candidate compound that fits the hypothesis, and determining if the candidate compound promotes a wild-type activity of a protein of the p53 family.

Another aspect of the invention is a composition comprising a complex of a protein of the p53 family and a non-peptide compound that interacts with the protein and promotes a wild type activity of the protein.

In still another aspect, the invention provides a method of screening for compounds that promote a wild-type activity of a protein of the p53 family. In a preferred aspect, the method comprises assaying for compounds that interact with the p53 DNA binding domain (DBD), and measuring the conformation of the p53 DBD in the presence of the compound. However, the invention also contemplates the use of full length and partial proteins of the p53 family in such methods of screening. In a particular embodiment, the assaying and measuring steps are performed simultaneously. Compounds discovered to promote a wild-type activity in a mutant form of a protein of the p53 family are optionally *screened in vivo* for their ability to halt or repress tumor growth. Another aspect of the invention is a method of drug discovery by screening organic non-peptide compounds for specific interaction with the p53 DBD.

The success of the present invention at identifying compounds that promote wild-type activity in a mutant or wild-type protein of the p53 family demonstrates that the methods of the invention are widely applicable to drug discovery for a class of diseases that are induced by conformationally defective or unstable proteins. Examples of such protein targets include pp60^{src}, ubiquitin activating enzyme E1, cystic fibrosis transmembrane conductance regulator, hemoglobin, prion proteins, serpins, and beta-amyloid protein.

### IV. Brief Description of the Figures

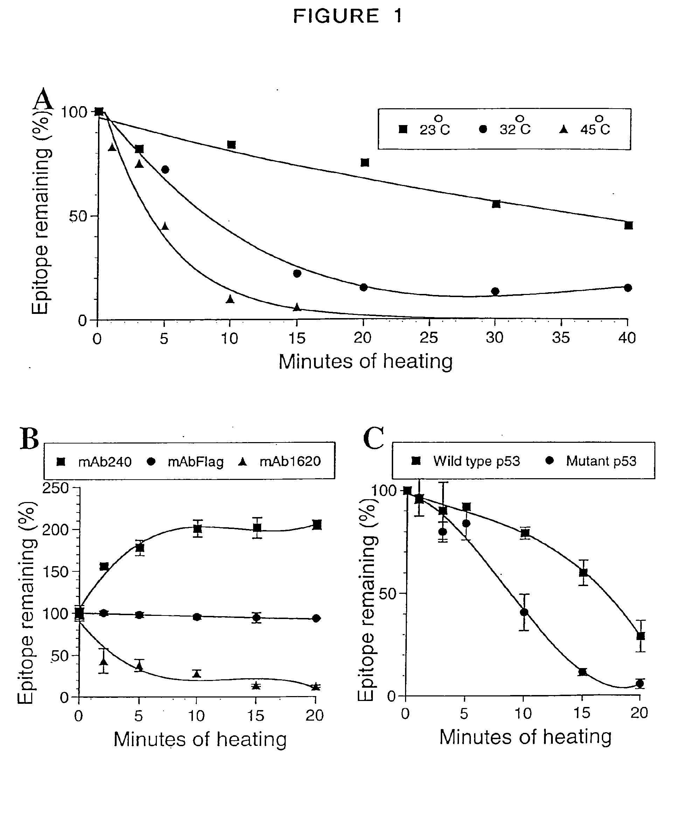

**Figure 1. Modulation of conformation-dependent epitopes on p53 DBD.** p53 DBD was immobilized in microtiter wells and incubated at elevated temperatures. An ELISA assay determined the percent of epitope for mAb1620 remaining in heated wells as compared to control wells which were maintained on ice. Figure 1A: 0.5 ng of wild-type p53 DBD was incubated and the remaining epitope for mAb1620 is shown as percent of the unheated control. Standard deviations were <10%. Figure 1B: 1.25 ng of FLAG-tagged p53 DBD was immobilized, heated at 45°C, and the remaining epitopes for anti-FLAG, mAb1620, and mAb240 were shown as percent of unheated control. Figure 1C: 1.0 ng of wild-type and position 143 mutant p53 DBD, which displayed approximately equal levels of the epitope for mAb1620, were heated at 37°C and the stability of the epitope was monitored as percent of unheated controls. Error bars are the standard deviation for 4 replicates.

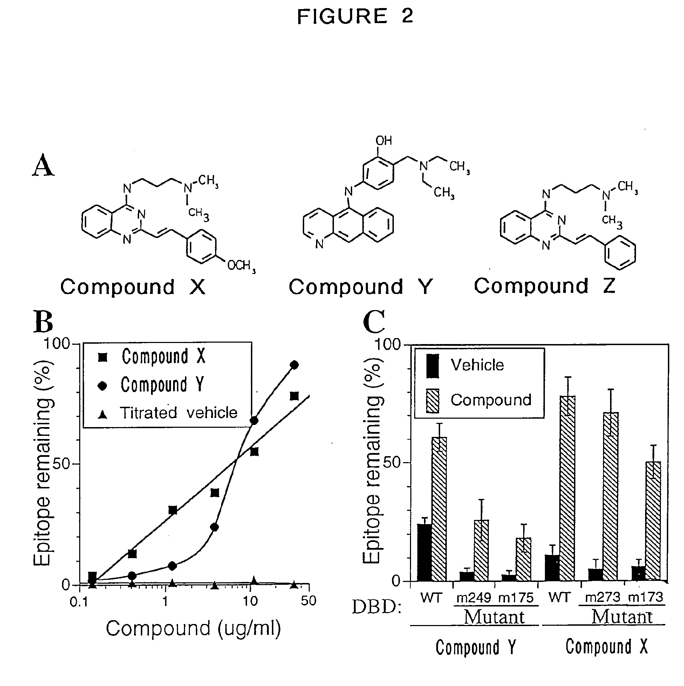

**Figure 2. Stabilization of the 1620 epitope on mutant p53 DBD.** Figure 2A: Representative compounds, designated Compound X, Compound Y and Compound Z, that promoted the conformational stability of p53. Figure 2B: 1 ng of wild-type p53 DBD was immobilized and heated at 45°C for 30 minutes in the presence of compounds or the equivalent concentration of the DMSO vehicle. The remaining epitope for mAb1620 is shown as percent of unheated control. Figure 2C: Wild-type and mutant p53 DBD preparations, with nearly equal levels of epitope for mAb1620 (within 10%), were immobilized and heated at 37°C for 30 minutes in the presence of compound or the vehicle. The remaining epitope for mAb1620 is shown as percent of unheated controls. Error bars are the standard deviation for 4 replicates.

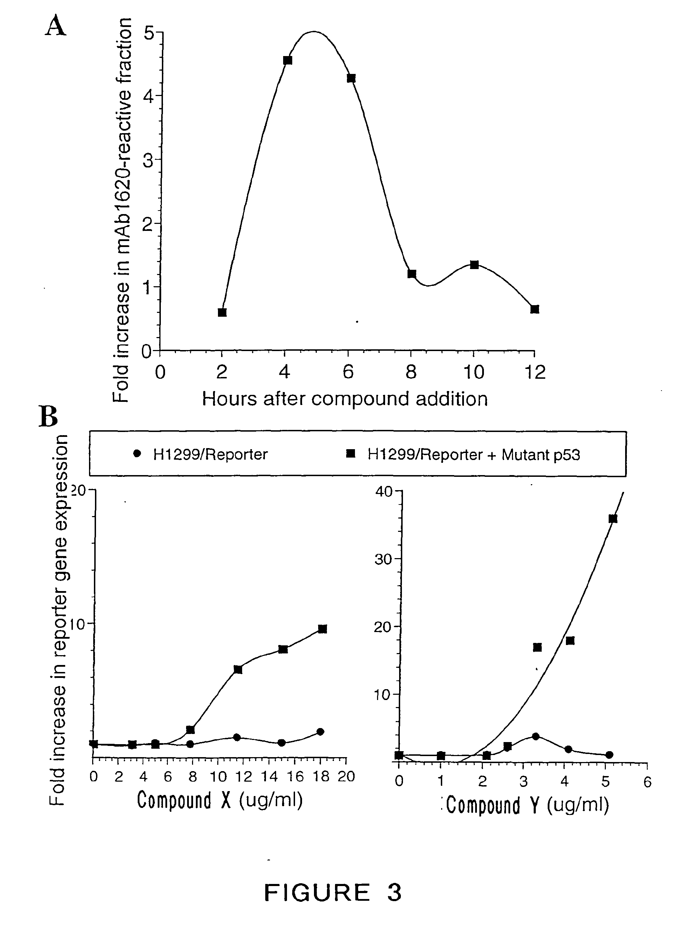

**Figure 3. Modulation of p53 conformation and transcription activity in cells with mutant p53.** Figure 3A: H1299 transfectants that expressed position 173 mutant p53 were treated with 16.5 ug/ml Compound X in culture. Cell lysates were normalized for minor variations in total p53 protein using Western blots with the pan p53 antibody, mAbDO-1, and amount of p53 that displayed the epitope for mAb1620 was determined in an ELISA assay. The increase in the 1620-positive p53 fraction was corrected for the fraction of 1620-positive p53 in untreated cells. Figure 3B: Matched H1299 transfectants with a luciferase reporter gene (H1299/Reporter) or with the reporter gene and the position 173 mutant p53 (H1299/Reporter + Mutant p53) were treated in microtiter wells for 16 hours. Induced expression of the luciferase reporter gene, which is indicative of wild-type p53 function, was corrected for the basal level of expression in the absence of compound. Values represent the average of 4 replicates.

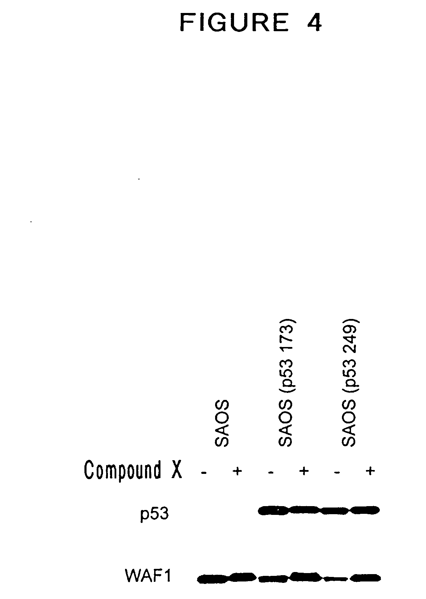

**Figure 4. Induction of WAF1 expression in cells with mutant p53.** Saos-2 cells expressing transfected mutant p53 proteins (position 173 or position 249) were treated in culture with 16.5 ug/ml Compound X for 16 hours. Cell lysates were normalized for total protein and analyzed on Western blots. The top portion of the blot was probed with mAbDO-1 for total p53 and the bottom portion of the same blot was probed with an antibody directed to WAF1.

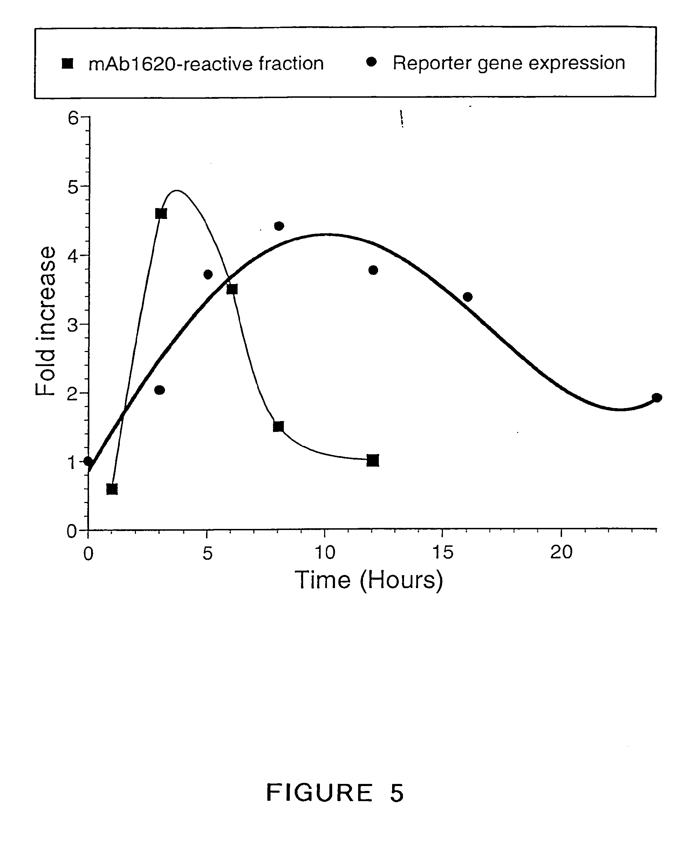

**Figure 5. Promotion of p53 conformational stability and function in tumors.** Mice harboring subcutaneous tumors derived from H1299/Reporter + Mutant p53 cells were given a single 100 mg/kg intra peritoneal injection of Compound X and duplicate tumor lysates were normalized for total p53 content based on densitometric scans of Western blots with mAbDO-1. The amount of p53 that displayed the epitope for mAb1620 was determined in an ELISA assay and the increase in the 1620-positive p53 fraction was corrected for the fraction of 1620-positive p53 in lysates from untreated tumors. Tumor lysates were also analyzed for luciferase expression to assess the enhancement of p53 transcription activity. Luciferase expression was normalized for protein concentration and compared to lysates from untreated tumors.

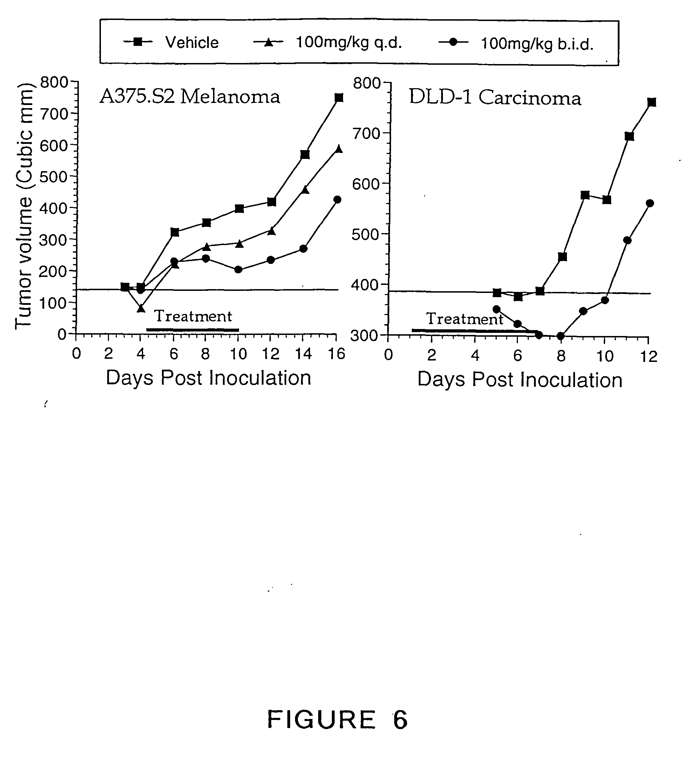

**Figure 6. Suppression of tumor xenografts expressing mutated p53.** Mice were inoculated with tumor cells and treated by intra peritoneal injections of Compound X or vehicle as indicated. The compound was administered for seven days at once daily (q.d.) or at 12 hour intervals (b.i.d.). Vehicle treated mice received injections at 12 hr intervals. Tumor volume was determined by measurement of tumor diameter in two dimensions and is averaged for 5-7 mice in each group. Dotted lines represent initial tumor volume when treatment was initiated.

### V. Detailed Description of the Invention

Loss of function in the tumor suppressor gene product p53 can lead to the uncontrolled proliferation and/or loss of apoptosis observed in many different types of cancers. Even if p53 is not mutated in a cancer cell, promoting wild-type p53 activity in such a cell can inhibit the cancerous phenotype. The invention demonstrates, for the first time, that organic non-peptide compounds can interact with a protein of the p53 family to stabilize functional conformation therein. Accordingly, such compounds have important use as pharmaceuticals for the treatment of all kinds of cancer.

Thus, in one aspect, the invention provides a method of promoting a wild-type activity in a mutant form of a human protein of the p53 family, wherein one or more functional activities of the protein are at least partially impaired by the inability of the protein to maintain a functional conformation under physiological conditions, the method comprising the steps of contacting the mutant protein with an organic non-peptide compound that is capable of binding to one or more domains in the mutant protein under physiological conditions and stabilizing a functional conformation therein, and permitting the stabilized protein to interact with one or more macromolecules that participate in the wild type activity. The mutant human protein of the p53 family can be a mutant p53, p63 or p73 protein. In preferred embodiments, the organic, non-peptide compound interacts with p53, and even more preferably, with the DNA binding domain of p53.

The invention also provides, in another embodiment, a method of treating a human subject for a disease state associated with expression of a mutant protein of the p53 family that has one or more diminished wild-type activities, comprising the steps of administering to the subject an organic non-peptide compound that is capable of binding to one or more domains in the mutant protein under physiological conditions, and stabilizing a functional conformation therein; and permitting the stabilized protein in the patient to interact with one or more macromolecules that participate in the wild-type activity.

In yet another embodiment, the invention provides a method of treating a human subject for cancer comprising the steps of: administering to the subject an organic non-peptide compound that is capable of binding to one or more domains of a human protein of the p53 family under physiological conditions, and stabilizing a functional conformation therein, and permitting the stabilized protein to interact with one or more macromolecules that participate in a wild-type activity of the protein. The human protein of the p53 family that is stabilized in the methods of the invention can be a wild-type or a mutant protein, for example, p53, p63 or p73.

Although proteins of the p53 family are mutant in a variety of cancers, nonetheless in some cancers or cancer cell types the structure or function of a protein of the p53 family (p53 itself has received the most study) is altered even though the involved cells retain a wild-type encoding allele. For example, see Kaelin, 1999, *supra*, for a discussion of virus-associated cancers wherein a viral protein degrades p53 protein, or p53 is inactivated or degraded by, for example, the expression products of oncogenes. Given the importance of proteins of the p53 family in cell regulatory processes, it will be apparent that the compounds of the invention are also useful to stabilize functional conformations of non-mutant p53 family members under physiological conditions in cells where the lifetime and/or structure and/or activity of such proteins is normal. Thus, the compounds of the invention are useful in the treatment of cancers where the function of p53 protein, and the like, is not substantially affected by the presence of the cancerous state, and also in the treatment of tissues expressing pre-cancerous cells whose abnormalities do not yet detectably extend to abnormal p53 (or p53 family member) function, lifetime or structure. Additionally, by further stabilizing (for example, causing an increased lifetime) proteins of the p53 family in healthy cells that are adjacent to sites of malignancy, or which otherwise come in contact with malignant cells in the body, the spread of cancers can be controlled. The compounds of the present invention are also useful in this regard.

According to the practice of the invention, a protein of the p53 family is defined as a mammalian p53, p63, or p73; and/or a protein that possesses a domain, all having at least 50%, more preferably 80%, of amino acid sequence homology to one or more of (1) the N-terminal domain required for transcriptional activation, (2) the DNA-binding domain, or (3) the oligomerization domain of a mammalian p53, p63, or p73, wherein said homology is measured by any of the recognized algorithms BLASTP v. 2.0 (www.ncbi.nlm.nih.gov) (Altschul et al., 1990, J. of Molec. Biol., 215:403-410, "The BLAST Algorithm; Altschul et al., 1997, Nuc. Acids Res. 25:3389-3402), and W.U.-BLAST-2.0 (available from Washington University, St. Louis, MO, USA), and wherein said protein evidences at least one function that is recognized in the art as characteristic also of p53, p63, or p73 (e.g., for example, capability of activating p53 responsive promoters and induce apoptosis; for discussion of art-recognized properties, see Kaelin, 1999; Yang *et al*., 1998; and Yoshikawa *et al*., 1999, cited above ). For a general discussion of the procedure and benefits of the BLAST, Smith-Waterman and FASTA algorithms *see* Nicholas et al., 1998, "A Tutorial on Searching Sequence Databases and Sequence Scoring Methods" (www.psc.edu) and references cited therein.

Compounds that stabilize the wild-type conformation of a protein of the p53 family are compounds that, when in contact with a protein of the p53 family, promote or restore a wild-type activity of the protein such as DNA binding affinity or the capacity to interact with any macromolecule to effect a normal function of the protein of the p53 family. Other wild-type activities of p53 include but are not limited to transcriptional activation activity (e.g., WAF1 induction), cell cycle arrest, and apoptosis triggering.

In yet another aspect, the invention includes the use of the compounds of the invention to inhibit tumor growth and/or treat cancer. A particular advantage of the invention is that the compounds so identified using the methods herein have been shown to stabilize the active conformation of not only wild-type p53 DBD and the mutant p53 DBD used in the screens, but also other mutant p53s and p53 DBDs. Therefore, the compounds so identified have broad applicability in treating varied cancers.

The present invention also provides a novel way of screening for compounds that promote the wild-type conformation of a protein of the p53 family and can restore wild-type activity to mutant proteins of the p53 family. Compounds identified using the methods of the invention are useful for treating diseases such as cancer that are associated with defects in activity of proteins of the p53 family.

The methods of the invention entail screening compounds for those that interact directly with a protein of the p53 family. Such methods can use a full length protein of the p53 family (mutant or wild-type) for screening purposes, or a deletion derivative containing at least the DBD and optionally the N terminal and/or C terminal domains. However, in a preferred aspect of the invention, the screens make use of a polypeptide fragment of a protein of the p53 family that contains only the DBD without the intact N or C terminal domains. Accordingly, for purposes of this Application, the term "the DNA binding domain" or "the DBD" is understood to include just the DBD of a protein of the p53 family, without an intact N or C terminus (unless indicated otherwise). Such DBD domains may, however, be fused to heterologous polypeptides depending upon the assay format (e.g., a FLAG epitope or a glutathione-S-transferase protein). Additionally, rather than merely removing a negative regulatory effect on DNA binding, the methods and compounds of the invention promote enhanced conformational stability of both wild-type and mutant proteins of the p53 family.

Accordingly, in one aspect illustrated below by way of a non-limiting working example, the invention provides a method of screening for compounds that specifically interact with the p53 DBD, and measuring the conformation of the p53 DBD in the presence of the test compound. Optionally, the p53 DBD is a mutant p53 DBD. However, wild-type p53 DBD is easier to overproduce in large quantities. Although the screening assay can be performed in a cell-based format, for high-throughput screens specific to compounds that target the p53 DBD, an *in vitro* based assay is most direct and desired. Compounds identified in an initial screen against the p53 DBD can be further tested for their effects on the function of intact p53 (including p53 missense mutants). Compounds identified using these methods are also within the scope of the invention.

For purposes of the instant invention, assays for compounds that interact with the DNA binding domain of a protein of the p53 family are designed such that compounds uncovered are those that specifically target the DBD and not other domains of the protein. For example, a compound that specifically "interacts with" or "acts on" the DBD need not necessarily bind stably to the DBD (although it may); it is sufficient for the compound to have some effect on the conformation of a protein of the p53 family in the presence of the compound. Accordingly, compounds may be first screened for interaction with the DBD, and then assayed for their effect on conformation, or these two screening steps may be performed simultaneously by using a conformational change in the presence of the compound to also detect interaction with the DBD.

The term specific interaction in this application is used to exclude unspecific forms of binding including the type known to occur between hydrophobic compounds and proteins through nonselective hydrophobic interactions. The term specific interaction is further used to distinguish the properties of the compounds of this invention from compounds that affect protein thermostability by changing the chemical properties of the bulk solvent. Such molecules excluded from the scope of this aspect of the invention therefore include thermostabilizing agents such as glycerol, trimethylamine ―oxide, and deuterated water. Compounds that specifically interact with a protein of the p53 family will show an effect at much lower concentrations than such bulk solvents or non-specific hydrophobic interactions. For example, glycerol is effective at 600 mM. However, effects of compounds that specifically interact with a protein of the p53 family will be observed at concentrations of the compound lower than 1 mM, preferably lower than 100 micomolar, and more preferably lower than 10 micromolar in *in vitro* or cell-based assays.

In connection with the practice of the invention, the following definitions will generally apply. The term "alkyl", as used herein, unless otherwise indicated, includes saturated monovalent hydrocarbon radicals having straight, branched or cyclic moieties or combinations thereof. Similarly, the terms "alkenyl" and "alknyl" define hydrocarbon radicals having straight, branched or cyclic moities wherein at least one double bond, or at least one triple bond, respectively, is present. Such definitions also apply when the alkyl, alkenyl or alkynyl group is present within another group, such as alkoxy or alkylamine. The term "alkoxy", as used herein, includes O-alkyl groups wherein "alkyl" is as defined above. The term "halo", as used herein, unless otherwise indicated, includes fluoro, chloro, bromo or iodo.

For convenience of description, the term (C₃-C₁₀) cycloalkyl when used herein refers to both cycloalkyl and cycloalkenyl groups, having zero or optionally one or more double bonds, such as cyclopropyl, cyclobutyl, cyclopentyl, cyclopentenyl, cyclohexyl, cyclohexenyl, 1,3-cyclohexadiene, cycloheptyl, cycloheptenyl, bicyclo[3.2.1]octane, norbornanyl, and the like. (C₃-C₁₀)heterocycloalkyl when used herein refers to pyrrolidinyl, tetrahydrofuranyl, dihydrofuranyl, tetrahydropyranyl, pyranyl, thiopyranyl, aziridinyl, oxiranyl, methylenedioxyl, chromenyl, isoxazolidinyl, 1,3-oxazolidin-3-yl, isothiazolidinyl, 1,3-thiazolidin-3-yl, 1,2-pyrazolidin-2-yl, 1,3-pyrazolidin-1-yl, piperidinyl, thiomorpholinyl, 1,2-tetrahydrothiazin-2-yl, 1,3-tetrahydrothiazin-3-yl, tetrahydrothiadiazinyl, morpholinyl, 1,2-tetrahydrodiazin-2-yl, 1,3-tetrahydrodiazin-1-yl, tetrahydroazepinyl, piperazinyl, chromanyl, etc. One of ordinary skill in the art will understand that the connection of said (C₃-C₁₀)heterocycloalkyl rings is through a carbon or a sp³ hybridized nitrogen heteroatom.

(C₅-C₉)heteroaryl when used herein refers to furyl, thienyl, thiazolyl, pyrazolyl, isothiazolyl, oxazolyl, isoxazolyl, pyrrolyl, triazolyl, tetrazolyl, imidazolyl, 1,3,5-oxadiazolyl, 1,2,4-oxadiazolyl, 1,2,3-oxadiazolyl, 1,3,5-thiadiazolyl, 1,2,3-thiadiazolyl, 1,2,4-thiadiazolyl, pyridyl, pyrimidyl, pyrazinyl, pyridazinyl, 1,2,4-triazinyl, 1,2,3-triazinyl, 1,3,5-triazinyl, pyrazolo[3,4-b]pyridinyl, cinnolinyl, pteridinyl, purinyl, 6,7-dihydro-5H-[1]pyrindinyl, benzo[b]thiophenyl, 5, 6, 7, 8-tetrahydro-quinolin-3-yl, benzoxazolyl, benzothiazolyl, benzisothiazolyl, benzisoxazolyl, benzimidazolyl, thianaphthenyl, isothianaphthenyl, benzofuranyl, isobenzofuranyl, isoindolyl, indolyl, indolizinyl, indazolyl, isoquinolyl, quinolyl, phthalazinyl, quinoxalinyl, quinazolinyl, benzoxazinyl, and the like. One of ordinary skill in the art will understand that the attachment of a (C₅-C₉) heteraryl group to the rest of a structure is generally without limitation, that is, through a carbon atom or an sp² hybridized heteroatom. Similarly, phenyl and naphthyl are representative of (C₆-C₁₀)aryl.

When, in a drawing, a bond is depicted but no identification is made as to the group placed at the distal end thereof, a methyl group is intended as is conventionally recognized. In the absence of any bond being depicted, the position is occupied by hydrogen, if valence permits, as is readily understood in the art. Thus the depiction, R- O ― means R- O ― CH₃.

### A. Compounds of the Invention That Promote Wild-type Activity in A Protein of the p53 Family

The organic non―peptide compounds of the invention can be any type of compound that, when exposed to a wild type or mutant protein of the p53 family, promote the wild type activity of the protein. Preferred compounds are relatively small (as compared to typical proteins of 50 to 150 kD) organic compounds. The present invention provides, for the first time, such compounds which are not peptides, and more particularly, not antibodies, yet which specifically interact with p53 and thereby stabilize a wild-type conformation of the p53 DBD or p53 protein. Organic compounds that are not peptides are particularly useful as pharmaceuticals for a variety of reasons. For example, non-peptide compounds are much less immunogenic than peptides, and more easily absorbed into the body through a mucosal or other cell layer barrier, and may be less labile.

In one aspect, active compounds discovered by the methods of the invention can be defined as a compound containing both a hydrophobic group (e.g., a planar polycyclic) and a cationic group (preferably an amine) joined together by a linker of a specific length. Benzimidazole, benzoquinoline, phenothiazine, and styrylquinazoline in the hydrophobic position are preferred.

Active cationic groups are both secondary and tertiary amines, including but not limited to dimethylamine, diethyl amine, diethanol amine, methyl amine, methyl piperazine, and morpholine. Certain larger amines were correspondingly more active when tested in the phenothiazine hydrophobic series; accordingly, a larger amine is preferred in this situation. Positively charged groups in the cationic position are active and preferred (see Table 1, *infra*.).

With respect to this aspect of the invention, the spacing between the hydrophobic and cationic groups should be at least a propyl length; linkers shorter than a propyl length were substantially less effective under the particular conditions of assay (see Table 2 *infra*). Therefore, linkers having the length of approximately 3 to 5 carbon bonds are preferred (from 5 to 9 Angstroms, and more preferably 6 to 8 Angstroms), although compounds containing linkers the length of a propyl linker (around 6.5 Angstroms) are most active. Linkers longer than the length of a butyl linker resulted in compounds that were less effective under the particular conditions of assay than corresponding compounds with linkers the length of a butyl linker (Table 2). Even more preferred are branched linkers which retain the correct distance; such linkers were generally more active in this assay than the corresponding linear linker as long as they still maintained about the right linker length of between 5 and 9 Angstroms (and optimally around 6.5 Angstroms).

Accordingly, in one aspect, the compounds of the invention have the formula:

F¹-L-F²

and F' is selected from the group consisting of: 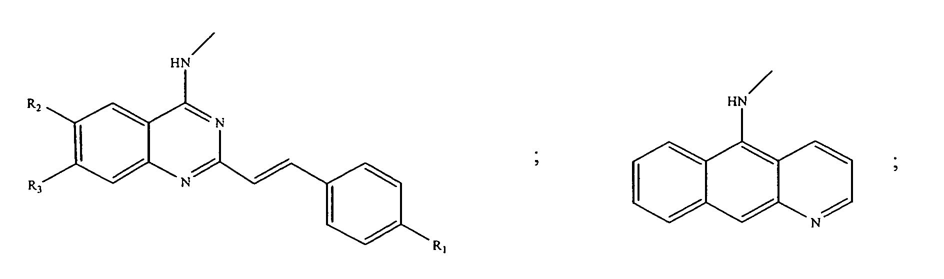 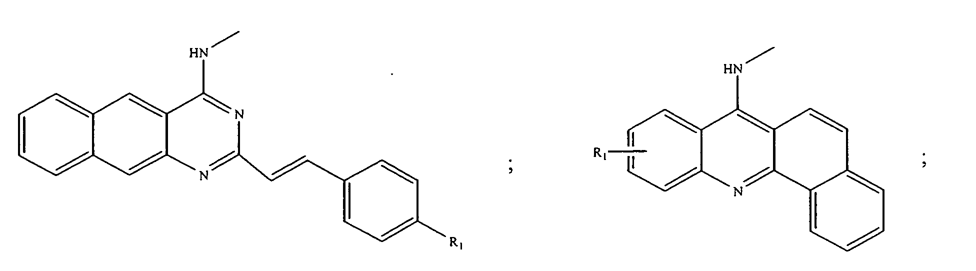 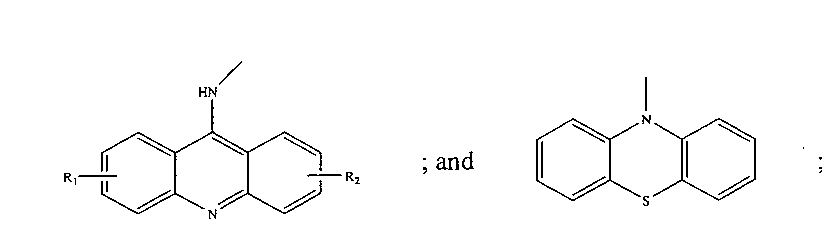 wherein
R₁, R₂, R₃ are the same or different and are independently selected from the group consisting of hydrogen, halogen, methoxy and nitro; L is a straight-chain or branched-chain alkyl having length from 5 to 9 Angstroms; and F² is a secondary or tertiary amine. In other aspects, F² is dimethyl amine, diethyl amine, diethanolamine, methyl piperazine or morpholin. For example, F² can be an amine selected from the group consisting of: 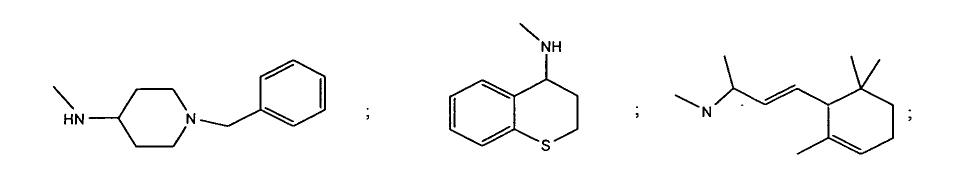 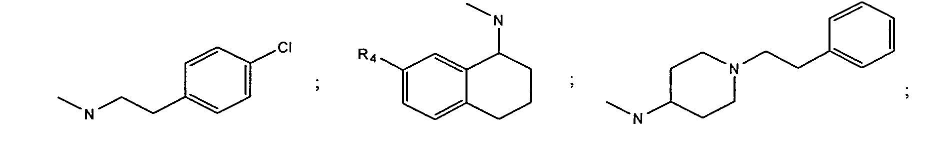 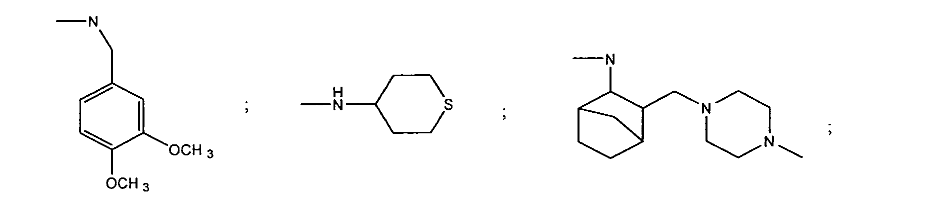 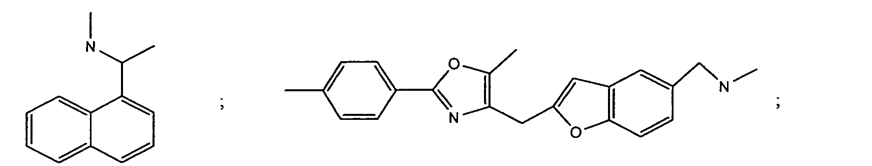 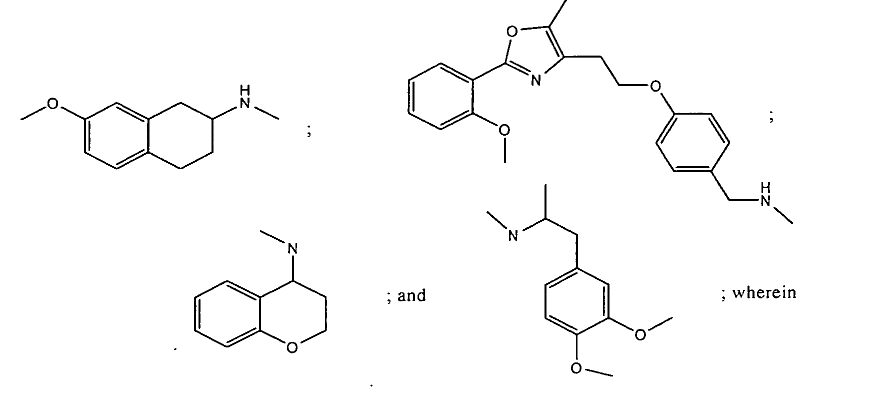 R₄ is -O-CH₂-CH₃ or H.

Provided below are chemical structures for various compounds of the invention. Each of these compounds was found to significantly enhance the stability of the conformation-sensitive epitope for p53 in at least one mutant p53 DBD at near physiological temperatures.

### 1. Acridines

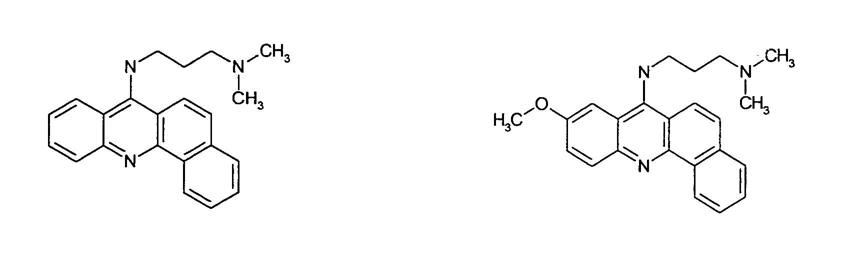

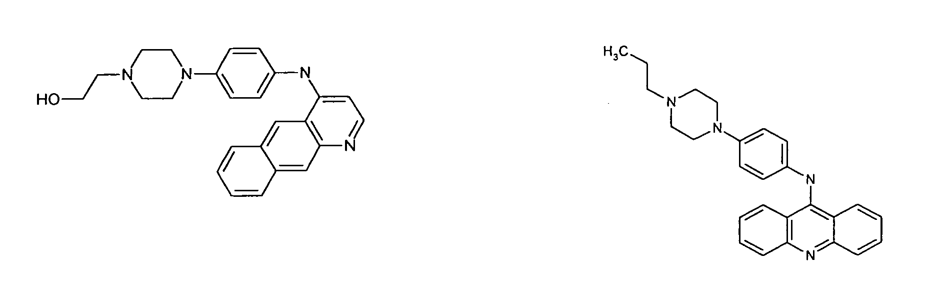

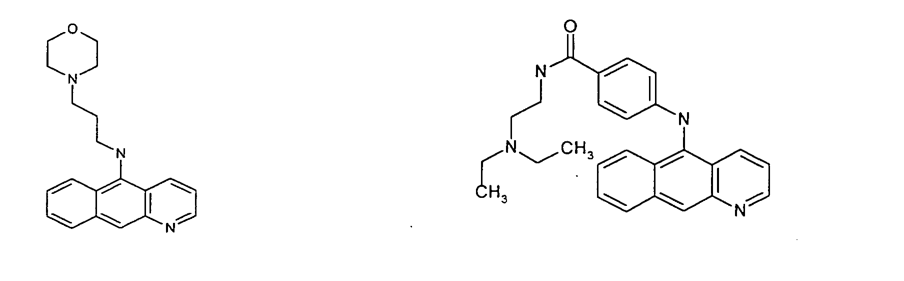

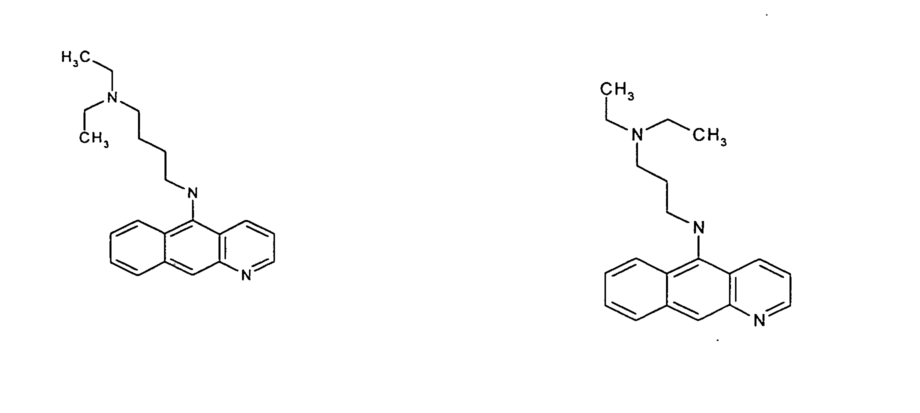

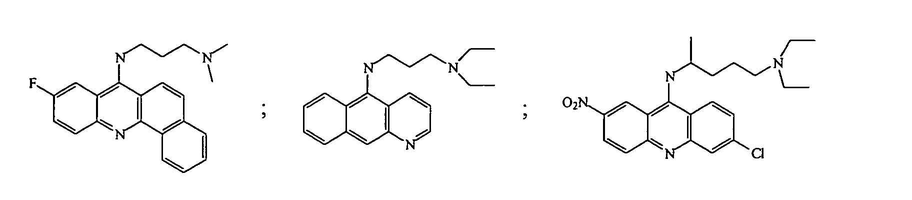

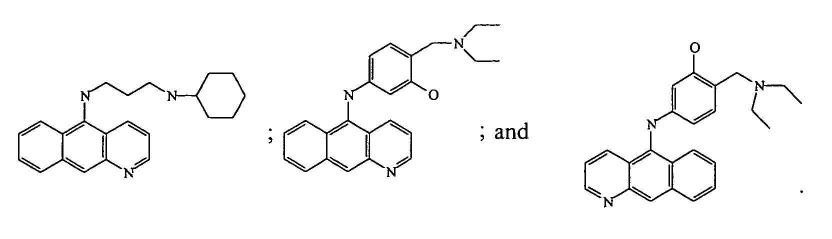

### 2. Quinazolines

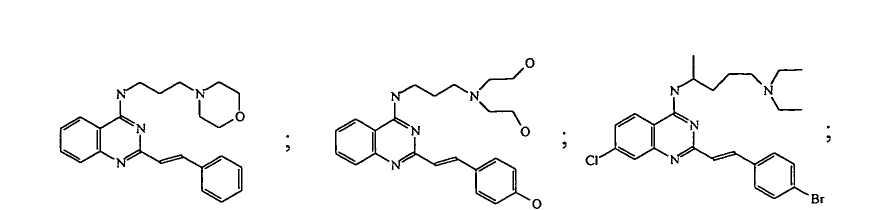

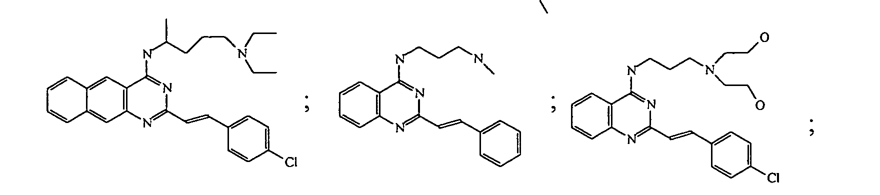

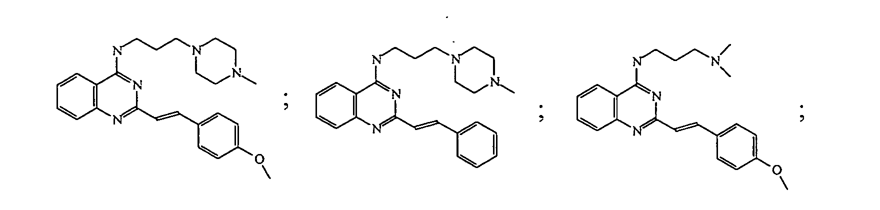

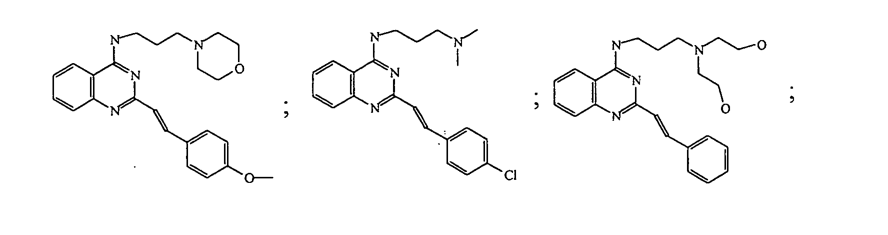

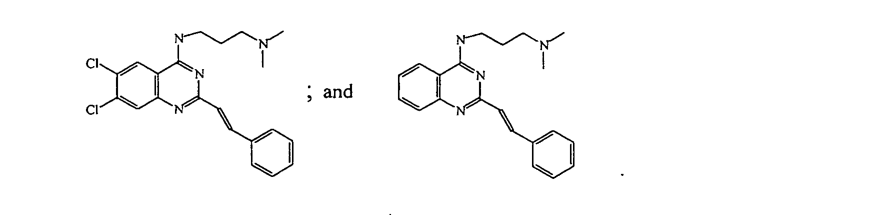

### 3. Phenothiazoles

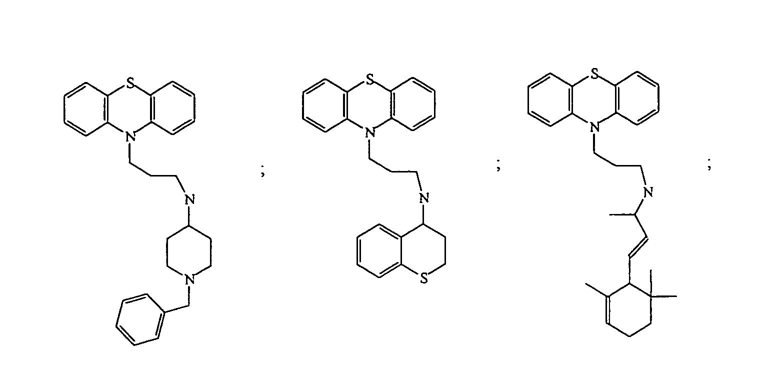

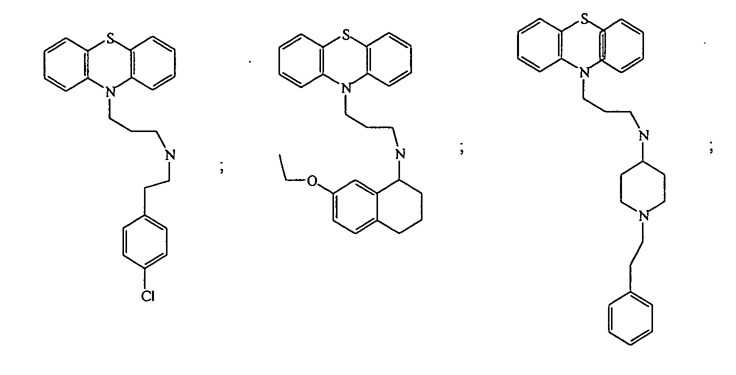

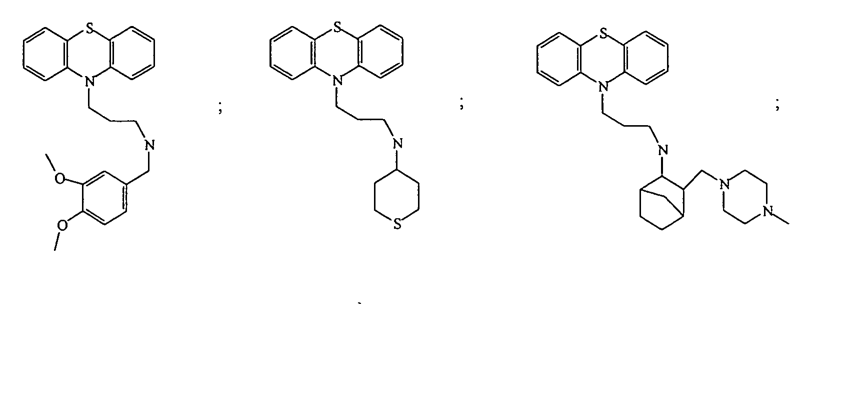

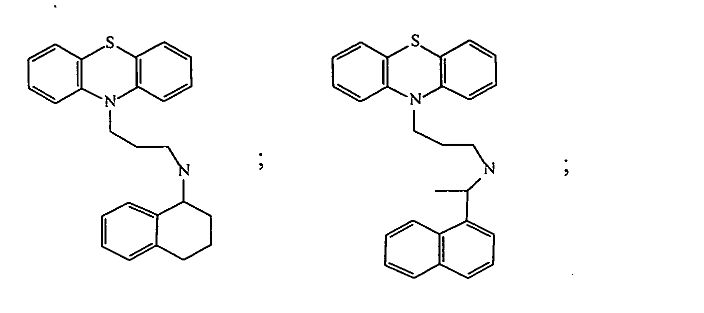

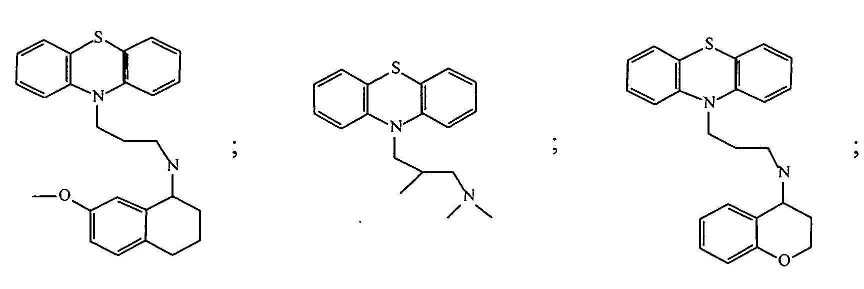

and

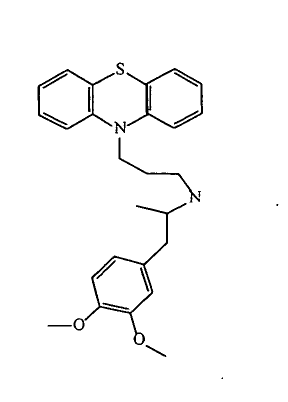

According to the general design principles described herein, the following groups of compounds are preferred in the practice of the present invention: 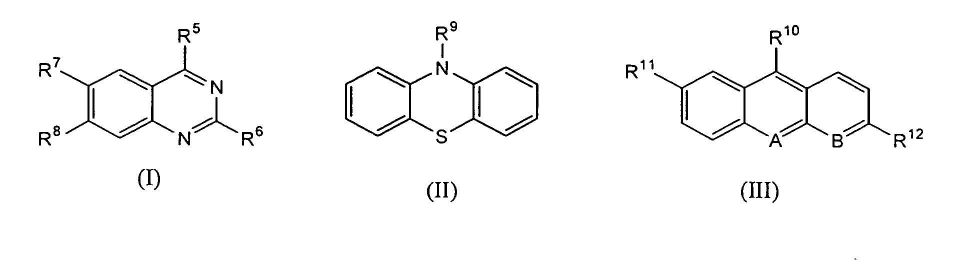 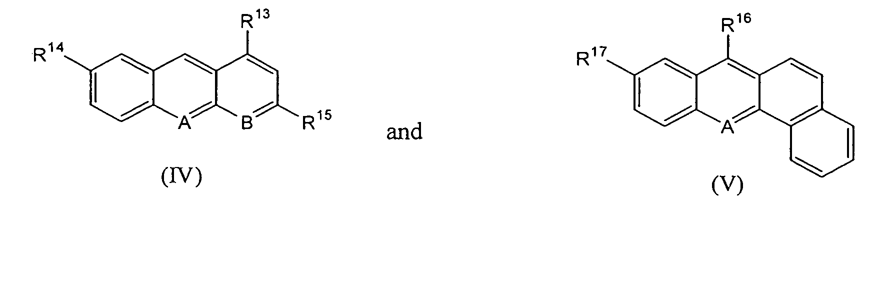 wherein, for group I, 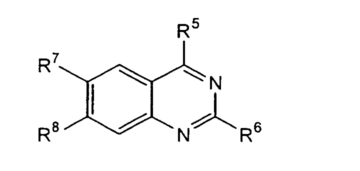 R⁵ is ―N―R¹⁸R¹⁹, where
R¹⁸ is H, (C₁-C₆)alkyl, or phenyl, and
R¹⁹ is H, (C₁-C₆)alkyl, (C₃-C₁₀)cycloalkyl, or phenyl, wherein said alkyl, cycloalkyl or phenyl group is optionally substituted with hydroxy, (C₃-C₈)cycloheteroalkyl,
―CON R¹⁸(CH₂)ₚNR²⁰R²¹, ―(CH₂)ₚ-(CHR²²)ₘ-(CH₂)ₙ-NR²⁰R²¹, or
―(CH₂)ₚ-(CHR²²)ₘ-(CH₂)ₙ―NR²⁰R²¹, wherein p is 0-5, m is 0-5, n is 0-5, R²² is hydroxy or (C₁-C₆)alkyl, and
R²⁰ and R²¹ are each, independently selected from:
(a) H, (C₁-C₁₂)alkyl, (C₃-C₁₂)cycloalkyl, (C₃-C₁₀)heterocycloalkyl, (C₆-C₁₀)aryl, (C₅-C₉)heteroaryl, (C₁-C₆)alkyl(C₆-C₁₂)aryl, wherein said groups are optionally substituted by one or more hydroxy, halo, amino, trifluoromethyl, (C₁-C₆)alkyl, (C₁-C₆)alkoxy, (C₁-C₆)alkyl(C₃-C₁₀)heterocycloalkyl, or (C₁-C₆)alkyl(C₆-C₁₀)aryl; or
(b) NR²⁰R²¹ taken together represent hydrogen, morpholine, or 4-(C₁-C₆) alkylpiperizine;
R⁶ is
(a) (C₁-C₆)alkyl or (C₂-C₈)alkenyl, each optionally substituted by one or more phenyl groups, or
(b) phenyl substituted by halo, (C₁-C₆)alkoxy; and
R⁷ and R⁸ are the same, or different, and are selected from H, nitro, (C₁-C₆)alkoxy, or halogen selected from fluoro, chloro, and bromo;
wherein, for group II, 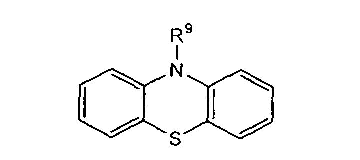 R⁹ is (C₁-C₆)alkyl, (C₃-C₁₀)cycloalkyl, or phenyl, wherein said alkyl, cycloalkyl or phenyl group is optionally substituted with hydroxy, (C₃-C₈)cycloheteroalkyl, ―CON R¹⁸(CH₂)ₚNR²⁰R²¹, ―(CH₂)ₚ-(CHR²²)ₘ-(CH₂)ₙ―NR²⁰R²¹, or
―(CH₂)ₚ-(CHR²²)ₘ-(CH₂)ₙ―NR²⁰R²¹, wherein p is 0-5, m is 0-5, n is 0-5, R²² is hydroxy or (C₁-C₆)alkyl, and
R²⁰ and R²¹ are each independently selected from H, (C₁-C₁₂)alkyl, (C₃-C₁₂)cycloalkyl, (C₃-C₁₀)heterocycloalkyl, (C₆-C₁₀)aryl, (C₅-C₉)heteroaryl, (C₁-C₆)alkyl(C₆-C₁₂)aryl, wherein said groups are optionally substituted by one or more hydroxy, halo, amino, trifluoromethyl, (C₁-C₆)alkyl, (C₁-C₆)alkoxy, (C₁-C₆)alkyl(C₃-C₁₀)heterocycloalkyl, (C₁-C₆)alkyl(C₅-C₉)heteroaryl, or (C₁-C₆)alkyl(C₆-C₁₀)aryl;
wherein, for group III, 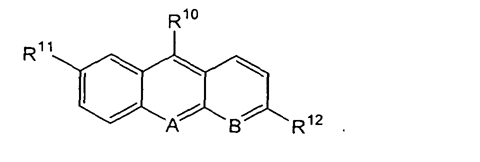 R¹⁰ is -N-R¹⁸R¹⁹, where
R¹⁸ is H, (C₁-C₆)alkyl, or phenyl, and
R¹⁹ is H, (C₁-C₆)alkyl, (C₃-C₁₀)cycloalkyl, or phenyl, wherein said alkyl, cycloalkyl or phenyl group is optionally substituted with hydroxy, (C₃-C₈)cycloheteroalkyl,
―CON R¹⁸(CH₂)ₚNR²⁰R²¹, ―(CH₂)ₚ-(CHR²²)ₘ-(CH₂)ₙ―NR²⁰R²¹, or
―(CH₂)ₚ-(CHR²²)ₘ-(CH₂)ₙ―NR²⁰R²¹, wherein p is 0-5, m is 0-5, n is 0-5, R²² is hydroxy or (C₁-C₆)alkyl, and
R²⁰ and R²¹ are each, independently selected from:
(a) H, (C₁-C₁₂)alkyl, (C₃-C₁₂)cycloalkyl, (C₃-C₁₀)heterocycloalkyl, (C₆-C₁₀)aryl, (C₅-C₉)heteroaryl, (C₁-C₆)alkyl(C₆-C₁₂)aryl, wherein said groups are optionally substituted by one or more hydroxy, halo, amino, trifluoromethyl, (C₁-C₆)alkyl, (C₁-C₆)alkoxy, (C₁-C₆)alkyl(C₃-C₁₀)heterocycloalkyl, (C₁-C₆)alkyl(C₅-C₉)heteroaryl, or (C₁-C₆)alkyl(C₆-C₁₀)aryl; or
(b) NR²⁰R²¹ taken together represent hydrogen, morpholine, or 4-(C₁-C₆) alkylpiperizine;
A and B are the same or different, and each represents carbon or nitrogen; and
R¹¹ and R¹² are the same, or different, and are selected from H, nitro, (C₁-C₆)alkoxy, or halogen selected from fluoro, chloro, and bromo;
wherein, for group IV, 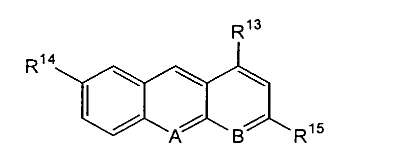 R¹³ is ―N―R¹⁸R¹⁹, where
R¹⁸ is H, (C₁-C₆)alkyl, or phenyl, and
R¹⁹ is H, (C₁-C₆)alkyl, (C₃-C₁₀)cycloalkyl, or phenyl, wherein said alkyl, cycloalkyl or phenyl group is optionally substituted with hydroxy, (C₃-C₈)cycloheteroalkyl,
―CON R¹⁸(CH₂)ₚNR²⁰R²¹, ―(CH₂)ₚ-(CHR²²)ₘ-(CH₂)ₙ―NR²⁰R²¹, or
―(CH₂)ₚ-(CHR²²)ₘ-(CH₂)ₙ―NR²⁰R²¹, wherein p is 0-5, m is 0-5, n is 0-5, R²² is hydroxy or (C₁-C₆)alkyl, and
R²⁰ and R²¹ are each, independently selected from:
(a) H, (C₁-C₁₂)alkyl, (C₃-C₁₂)cycloalkyl, (C₃-C₁₀)heterocycloalkyl, (C₁-C₆)alkyl(C₅-C₉)heteroaryl, (C₅-C₉)heteroaryl, (C₆-C₁₀)aryl, and (C₁-C₆)alkyl(C₆-C₁₀)aryl, wherein said groups are optionally substituted by one or more hydroxy, halo, amino, trifluoromethyl, (C₁-C₆)alkyl, (C₁-C₆)alkoxy, (C₁-C₆)alkyl(C₃-C₁₀)heterocycloalkyl, (C₁-C₆)alkyl(C₅-C₉)heteroaryl and (C₁-C₆)alkyl(C₆-C₁₀)aryl; or
(b) NR²⁰R²¹ taken together represent hydrogen, morpholine, or 4-(C₁-C₆) alkylpiperizine;
A and B are the same or different, and each represents carbon or nitrogen; and
R¹⁴ and R¹⁵ are the same, or different, and are selected from H, nitro, (C₁-C₆)alkoxy, or halogen selected from fluoro, chloro, and bromo; and
wherein, for group V, 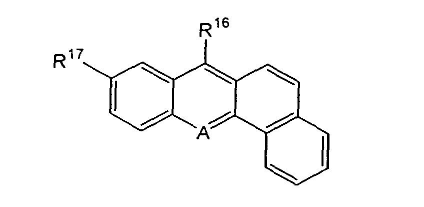 A is carbon or nitrogen;
R¹⁶ is ―N―R¹⁸R¹⁹, where
R¹⁸ is H, (C₁-C₆)alkyl, or phenyl, and
R¹⁹ is H, (C₁-C₆)alkyl, (C₃-C₁₀)cycloalkyl, or phenyl, wherein said alkyl, cycloalkyl or phenyl group is optionally substituted with hydroxy, (C₃-C₈)cycloheteroalkyl,
―CON R¹⁸(CH₂)ₚNR²⁰R²¹, ―(CH₂)ₚ-(CHR²²)ₘ-(CH₂)ₙ―NR²⁰R²¹, or
―(CH₂)ₚ-(CHR²²)ₘ-(CH₂)ₙ-NR²⁰R²¹, wherein p is 0-5, m is 0-5, n is 0-5, R²² is hydroxy or (C₁-C₆)alkyl, and
R²⁰ and R²¹ are each, independently selected from:
(a) H, (C₁-C₁₂)alkyl, (C₃-C₁₂)cycloalkyl, (C₃-C₁₀)heterocycloalkyl, (C₆-C₁₀)aryl, (C₅-C₉)heteroaryl, (C₁-C₆)alkyl(C₆-C₁₀)aryl, and (C₁-C₆)alkyl(C₅-C₉)heteroaryl, or wherein said groups are optionally substituted by one or more hydroxy, halo, amino, trifluoromethyl, (C₁-C₆)alkyl, (C₁-C₆)alkoxy, (C₁-C₆)alkyl(C₃-C₁₀)heterocycloalkyl, (C₁-C₆)alkyl(C₅-C₉)heteroaryl, or (C₁-C₆)alkyl(C₆-C₁₀)aryl; or
(b) NR²⁰R²¹ taken together represent hydrogen, morpholine, or 4-(C₁-C₆) alkylpiperizine; and
R¹⁷ selected from H, nitro, (C₁-C₆)alkoxy, or halogen selected from fluoro, chloro, and bromo.

Particularly preferred compounds of the invention include the following eleven compounds:

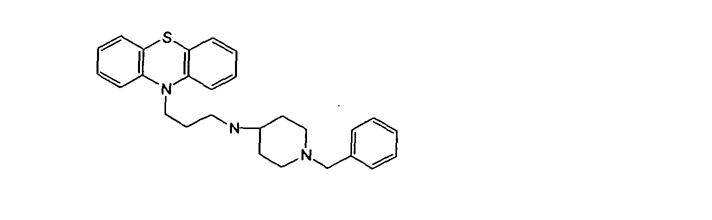

### (1-Benzyl-piperidin-4-yl)-(3-phenothiazin-10-yl-propyl)-amine

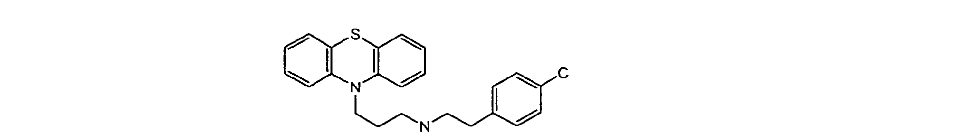

### [2-(4-Chloro-phenyl)-ethyl]-(3-phenothiazin-10-yl-propyl)-amine

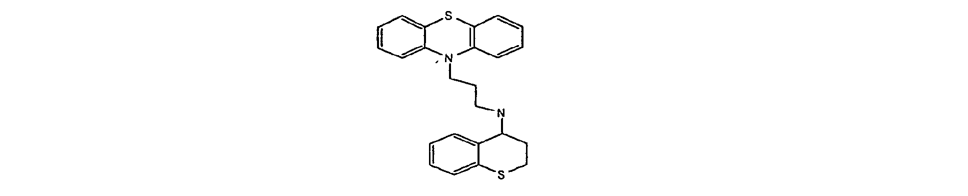

### (3-Phenothiazin-10-yl-propyl)-thiochroman-4-yl-amine

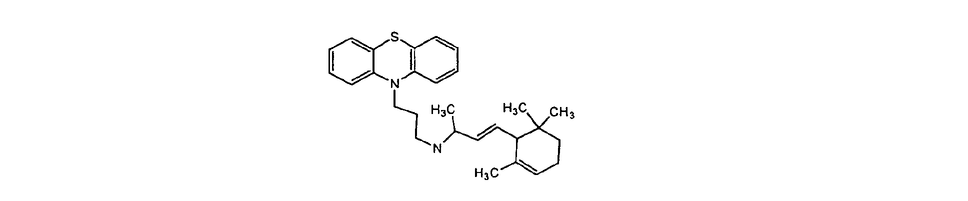

### [1-Methyl-3-(2,6,6-trimethyl-cyclohex-2-enyl)-allyl]-(3-phenothiazin-10-yl-propyl)-amine

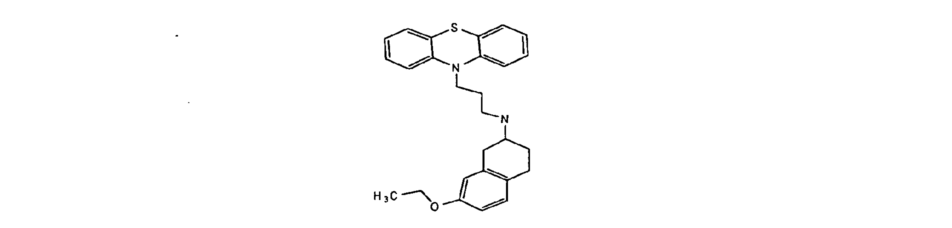

### (7-Ethoxy-1,2,3,4-tetrahydro-naphthalen-2-yl)-(3-phenothiazin-10-yl-propyl)-amine

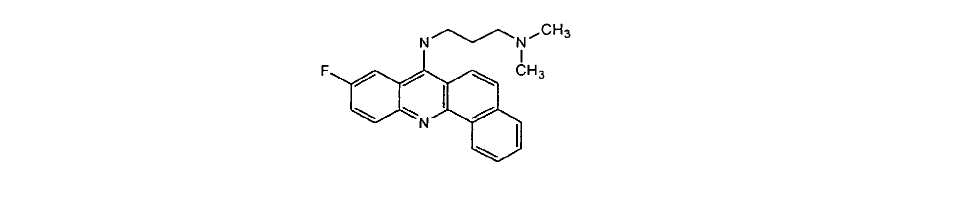

### N'-(9-Fluoro-benzo[c]acridin-7-yl)-N,N-dimethyl-propane-1,3-diamine

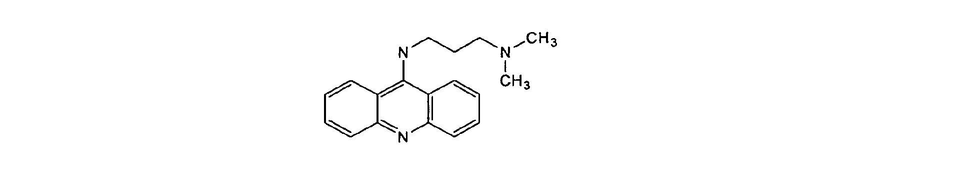

### N'-Acridin-9-yl-N,N-dimethyl-propane-1,3-diamine

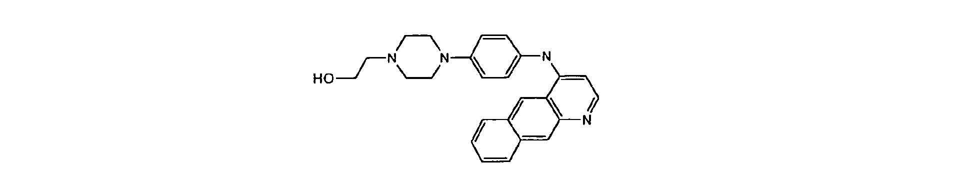

2-{4-[4-(Benzo[g]quinolin-4-ylamino)-phenyl]-piperazin-1-yl}-ethanol

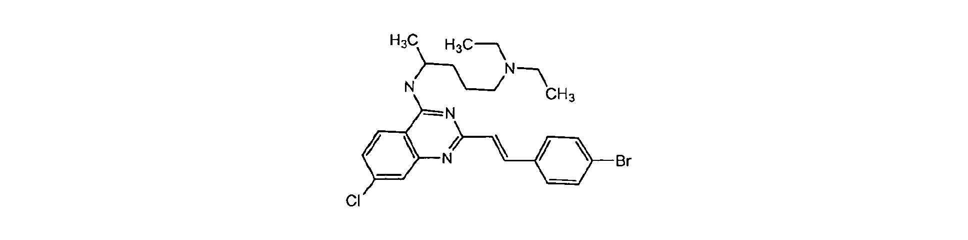

### N⁴-{2-[2-(4-Bromo-phenyl)-vinyl]-7-chloro-quinazolin-4-yl}-N¹,N¹-diethyl-pentane-1,4-diamine

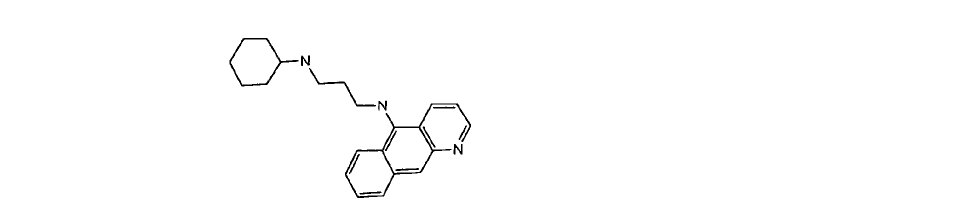

### N-Benzo[g]quinolin-5-yl-N'-cyclohexyl-propane-1,3-diamine

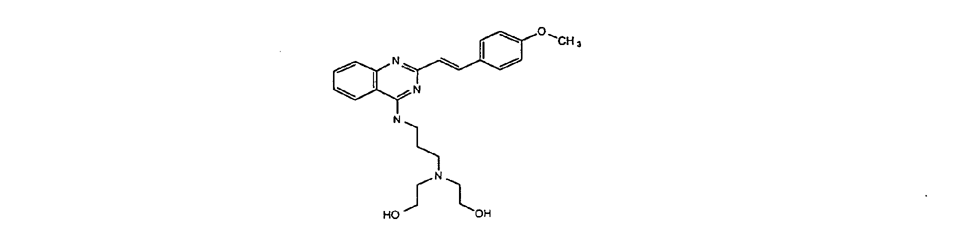

### 2-[(2-Hydroxy-ethyl)-(3-{2-[2-(4-methoxy-phenyl)-vinyl]-quinazolin-4-ylamino}-propyl)-amino]-ethanol.

The organic non-peptide compounds of the present invention can be synthesized using conventional techniques.

The compounds of the invention and for use in the methods of the invention also include prodrugs of compounds that promote a wild-type activity of a protein of the p53 family. Prodrugs are compounds that, when administered to a subject mammal (particularly a human), are converted in significant and effective quantities to the active molecule.

The compounds of the invention can be in the form of free acids, free bases or pharmaceutically effective salts thereof. Such salts can be readily prepared by treating a compound with an appropriate acid. Such acids include, by way of example and not limitation, inorganic acids such as hydroholic acids (hydrochloric, hydrobiomic, etc.), sulfuric acid, nitric acid, phosphoric acid, etc; and organic acids such as acetic acid, propanoic acid, 2-oxoproponoic acid, propandoic acid, butandoic acid, etc. Conversely, the salt can be converted into the free base form by treatment with alkali.

### B. Therapeutic Endpoints and Dosages

The compounds identified by the methods of the invention are useful for the treatment of diseases associated with conformationally unstable or misfolded proteins. Diseases associated with conformationally unstable or misfolded proteins are known and include cystic fibrosis (CFTR), Marfan syndrom (fibrillin), Amyotrophic lateral sclerosis (superoxide dismutase), scurvy (collagen), maple syrup urine disease (alpha-ketoacid dehydrogenase complex), osteogenesis imperfecta (typeI procollagen pro-alpha), Creutzfeldt-Jakob disease (prion), Alzheimer's disease (beta-amyloid), familial amyloidosis (lysozyme), cataracts (crystallins), familial hypercholecterolemia (LDL receptor), α1-antitrypsin deficiency, Tay-Sachs disease (beta-hexosaminidase), retinitis pigmentosa (rhodopsin), and leprechaunism (insulin receptor). Of course, the methods and compounds described herein are particularly useful in the treatment of cancers, and especially useful in the treatment of cancers associated with mutant p53 genes.

One of ordinary skill will appreciate that, from a medical practitioner's or patient's perspective, virtually any alleviation or prevention of an undesirable symptom associated with a disease condition, and in particular a cancerous condition (e.g., pain, sensitivity, weight loss, and the like) would be desirable. Additionally, with respect to a cancerous condition, any reduction in tumor mass or growth rate is desirable, as well as an improvement in the histopathological picture of the tumor. Thus, for the purposes of this Application, the terms "treatment," "therapeutic use, or "medicinal use" used herein shall refer to any and all uses of the claimed compositions which remedy a disease state or symptoms, or otherwise prevent, hinder, retard, or reverse the progression of disease or other undesirable symptoms in any way whatsoever.

An effective dosage and treatment protocol may be determined by conventional means, starting with a low dose in laboratory animals and then increasing the dosage while monitoring the effects, and systematically varying the dosage regimen as well. Animal studies, preferably mammalian studies, are commonly used to determine the maximal tolerable dose, or MTD, of bioactive agent per kilogram weight. Those skilled in the art regularly extrapolate doses for efficacy and avoiding toxicity to other species, including human.

Before human studies of efficacy are undertaken, Phase I clinical studies in normal subjects help establish safe doses. Numerous factors may be taken into consideration by a clinician when determining an optimal dosage for a given subject. Primary among these is the toxicity and half-life of the chosen heterologous gene product. Additional factors include the size of the patient, the age of the patient, the general condition of the patient, the particular cancerous disease being treated, the severity of the disease, the presence of other drugs in the patient, the *in vivo* activity of the gene product, and the like. The trial dosages would be chosen after consideration of the results of animal studies and the clinical literature.

As shown below by way of an actual working embodiment, a dose of 200 mg/kg/day was highly effective for inhibiting and/or regressing tumor growth in an animal model of a human cancer. Based on this result, a typical human dose of the compound Compound X for the treatment of a cancer is from 0.1 to 10 g /day injected i.v. or directly into the tumor mass or administered orally, depending upon the subject's condition. For a compound with a different level of efficacy and/or toxicity, these values would of course be altered accordingly. Additionally, doses can be given in two or more increments per day.

The compounds for use in the methods of the invention can also be formulated as a slow release implantation device for extended and sustained administration. Examples of such sustained release formulations include composites of bio-compatible polymers, such as poly(lactic acid), poly(lactic-co-glycolic acid), methylcellulose, hyaluronic acid, collagen, and the like. The structure, selection and use of degradable polymers in drug delivery vehicles have been reviewed in several publications, including, A. Domb *et al*., Polymers for Advanced Technologies 3:279-292 (1992). Additional guidance in selecting and using polymers in pharmaceutical formulations can be found in the text by M. Chasin and R. Langer (eds.), "Biodegradable Polymers as Drug Delivery Systems," Vol. 45 of "Drugs and the Pharmaceutical Sciences," M. Dekker, New York, 1990, and U.S. Patent No. 5,573,528 to Aebischer *et al*. (issued November 12, 1996).

Particularly where *in vivo* use is contemplated, the various biochemical components of the present invention are preferably of high purity and are substantially free of potentially harmful contaminants (e.g., at least National Food (NF) grade, generally at least analytical grade, and preferably at least pharmaceutical grade). To the extent that a given compound must be synthesized prior to use, such synthesis or subsequent purification shall preferably result in a product that is substantially free of any potentially toxic agents which may have been used during the synthesis or purification procedures.

For use in treating a cancerous condition in a subject, the present invention also provides in one of its aspects a kit or package, in the form of a sterile-filled vial or ampule, that contains a compound shown to be efficacious in the methods of the invention. In one embodiment, the kit contains a compound of the invention, such as Compound Y, Compound X or Compound Z, as an administration-ready formulation, in either unit dose or multi-dose amounts, wherein the package incorporates a label instructing use of its contents for the treatment of cancer. Alternatively, and according to another embodiment of the invention, the package provides a sterile-filled vial or ampule containing such a compound.

### C. Drug Discovery Methods

Each or all of the steps in screening compounds that interact with a protein of the p53 family, and particular a p53 DBD, and/or affect it's wild-type activity are amenable to high throughput assays for candidate compounds. High through-put screens are well known in the art and can be performed in any of a number of formats. For example, ELISAs, scintillation proximity technology, competitive binding assays and displacement binding assays are useful formats. Laboratory automation, including robotics technology, can vastly decrease the time necessary to screen large numbers of compounds and is commercially available from, for example, Tecan, Scitec, Rosys, Mitsubishi, CRS Robotics, Fanuk, and Beckman-Coulter Sagian, to name just a few companies. After candidate compounds are identified (or concurrently with their identification), secondary screens can be performed to determine the cellular and/or *in vivo* effects of the compounds on the activity of a protein of the p53 family.

### 1. Proteins of the p53 Family Targeted By The Methods and Compositions of the Invention

p53 is ubiquitous in all eukaryotic organisms. Accordingly, the p53 proteins and p53 DBD's for use in the methods and compositions of the invention can be from, or derived from, any eukaryotic cell including fungi (e.g., *Saccharomyces cerevisiae*), insects (e.g., *Drosophila)* and mammals (e.g., mouse and/or human), although human p53 proteins are preferred. Additional mammalian homologs of p53 with related structure and function, notably p63 and p73, have been identified; such proteins of the p53 family, and for example, their respective DBDs, can also be used in the methods and compositions of the invention. In addition, proteins of the p53 family (as herein defined) but yet to be discovered can also be used in the methods and compositions of the invention.

As noted above, the p53 protein contains at least three different domains: a transcriptional activation domain located at the amino terminus; the central DBD; and an oligomerization domain at the carboxyl terminus. Additionally, a negative-regulating domain appears in the carboxyl terminus of the protein. Most of the p53 missense mutations associated with human cancers occur in the DBD. The methods and compounds of the invention are directed at stabilizing the conformation of any such missense mutations. Particularly preferred targets are mutant p53s containing one or more of the so-called "hotspots" for mutation at residue positions 175, 245, 248, 249, 273 and 282 (all residue positions are given with respect to the human p53 sequence; the analogous residue position in p53 proteins from other organisms can be easily determined by homology alignment with the human sequence). Other common mutations in p53 occur at 132, 135, 138, 141, 143, 146, 151, 152, 154, 157, 158, 159, 163, 173, 176, 179, 186, 194, 196, 213, 220, 237, 238, 241, 242, 258, 266, 272, 278, 280, 281, 285 and 286; these are also targets for the invention. Further, the invention is illustrated below by way of working examples showing conformational stabilization of the following mutant p53 proteins: 143A, 173A, 175S, 241D, 249S and 273H.

Cancers associated with missense mutations in the p53 proteins, particularly in the DBD of p53 protein, include but are not limited to colorectal carcinoma, bladder carcinoma, hepatocellular carcinoma, ovarian carcinoma, lung carcinoma, breast carcinoma, squamous cell carcinoma in head and neck, esophageal carcinoma, thyroid carcinoma, and neurogenic tumors such as astrocytoma, ganglioblastoma and neuroblastoma. The above cancers, and others, are treatable by the methods and compounds of the invention.

The p53 DBD resides in approximately amino acids residues 100-300. A proteolysis-resistant core of residues 102 to 292 has been shown sufficient for DNA binding, and the p53 DBD crystal structure has been solved for residues 94 to 312 (Cho *et al*., 1994, Science 265, 346; Friend, 1994, Science 265, 334). Accordingly, for use in the methods of the invention, the N-terminus of the p53 DBD domain can begin from residue 50 to residue 110, and preferably starts somewhere between residues 94 and 102. The C-terminus of the p53 DBD can end at residue 286 to residue 340, and preferably ends between residue 292 to 312.

"Thermodynamically destabilized mutants of p53 " are mutants that do not retain one or more of the functional properties of p53 such as DNA binding at physiological temperatures (i.e., around 37°C), but regain such function(s) at lowered temperatures, or under other conditions. For example, all of the commonly encountered mutants retain the capacity to bind DNA *in vitro* at low temperature (Friedlander *et al*., 1996, *supra*.).

### 2. Assay Formats

### a. Binding Assay Formats

The principle of the assays used to identify compounds that simply bind to the the DBD of a protein of the p53 family involves preparing a reaction mixture of the DBD protein and the test compound under conditions and for a time sufficient to allow the two components to interact and bind, thus forming a complex which can be removed and/or detected in the reaction mixture. The DBD species used can vary depending upon the goal of the screening assay. For example, where compounds that interfere with a particular binding domain are sought, the full length protein of the p53 family containing that binding domain, the DBD itself, or a fusion protein containing DBD fused to a protein or polypeptide that affords advantages in the assay system (e.g., labeling, isolation of the resulting complex, etc.) can be utilized. The peptides derived from the DBD for use in this technique should comprise at least 6 consecutive amino acids, preferably 10 consecutive amino acids, more preferably 20 consecutive amino acids, even more preferably 30 or even 50 consecutive amino acids, or more, of the DBD.

The screening assays can be conducted in a variety of ways. For example, one method to conduct such an assay would involve anchoring the DBD protein, polypeptide, peptide or fusion protein or the test substance onto a solid phase and detecting DBD/test compound complexes anchored on the solid phase at the end of the reaction. In one embodiment of such a method, the DBD reactant may be anchored onto a solid surface, and the test compound, which is not anchored, may be labeled, either directly or indirectly. Any of a variety of suitable labeling systems can be used including but not limited to radioisotopes such as ¹²⁵I and ³²P, enzyme labeling systems that generate a detectable colorimetric signal or light when exposed to a substrate, and fluorescent labels. In another embodiment of the method, a DBD protein anchored on the solid phase is complexed with labeled antibody. Then, a test compound could be assayed for its ability to disrupt the association of the DBD/antibody complex.

In practice, microtiter plates may conveniently be utilized as the solid phase. The anchored component may be immobilized by non-covalent or covalent attachments. Noncovalent attachment may be accomplished by simply coating the solid surface with a solution of the protein and drying. Alternatively, an immobilized antibody, preferably a monoclonal antibody, specific for the protein to be immobilized may be used to anchor the protein to the solid surface. The surfaces may be prepared in advance and stored.

In order to conduct the assay, the non-immobilized component is added to the coated surface containing the anchored component. After the reaction is complete, unreacted components are removed (e.g., by washing) under conditions such that any complexes formed will remain immobilized on the solid surface. The detection of complexes anchored on the solid surface can be accomplished in a number of ways. Where the previously nonimmobilized component is pre-labeled, the detection of label immobilized on the surface indicates that complexes were formed. Where the previously nonimmobilized component is not pre-labeled, an indirect label can be used to detect complexes anchored on the surface; e.g., using a labeled antibody specific for the previously nonimmobilized component (the antibody, in turn, may be directly labeled or indirectly labeled with a labeled anti-Ig antibody).

In other embodiments, binding can be detected without making use of a direct or indirect label. For example, a biophysical property which alters when binding occurs can be assayed. A solid support system particularly advantageous for such screening is the BIAcore 2000™ system, available commercially from BIAcore, Inc. (Piscataway, NJ). The BIAcore™ instrument (http://www.biacore.com) uses the optical phenomenon of surface plasmon resonance (SPR) to monitor biospecific interactions in real-time. The SPR effect is essentially an evanescent electrical field that is affected by local changes in refractive index at a metal-liquid interface. A sensor chip made up of a sandwich of gold film between glass and a carboxymethyl dextran matrix to which the ligand or protein to be assayed is chemically linked. This sensor chip is mounted on a fluidics cartridge forming flow cells through which analyte compounds can be injected. Ligand-analyte interactions on the sensor chip are detected as changes in the angle of a beam of polarized light reflected from the chip surface. Binding of any mass to the chip affects SPR in the gold/dextran layer. This change in the electrical field in the gold layer interacts with the reflected light beam and alters the angle of reflection proportional to the amount of mass bound. Reflected light is detected on a diode array and translated to a binding signal expressed as response units (RU). As the response is directly proportional to the mass bound, kinetic and equilibrium constants for protein-protein interactions can be measured.

Alternatively, a reaction can be conducted in a liquid phase, the reaction products separated from unreacted components, and complexes detected.

### b. Methods for Measuring Conformation of a Protein of the p53 Family

Conformation of the protein of the p53 can be measured in any of a number of different ways. For example, antibodies can be used to probe conformation of the p53 DBD. Preferred methods of the invention use monoclonal antibodies that are specific for active (e.g., DNA binding) or inactive (thermodynamically destabilized, or misfolded or unfolded) conformations of p53 and/or p53 DBD. For example, mAb1620 recognizes an epitope on p53 DBD is tightly associated with the p53 protein's tumor suppressor activity. Ball *et al*., 1984, EMBO J. 3: 1485-1491; Gamble *et al*., 1988, Virology 162:452-458. Thus mAb 1620 will not bind the p53 DBD when it adopts an inactive conformation. Conversely, the epitope recognized by mAb 240 is exposed when p53 is inactivated by mutation or wild-type p53 is denatured (Bartek *et al*., 1990, Oncogene 5, 893-899; Stephen *et al*., 1992, J. Mol. Biol. 225, 577-83). Other monoclonal antibodies, known or yet to be discovered, that are conformation-specific can also be used in the methods of the invention. Such antibodies are useful because they can be easily adapted to high-throughput screens. Methods of making antibodies, including monoclonal antibodies, are well known in the art.

Other ways of measuring conformation of a protein of the p53 family such as p53 or a p53 DBD include but are not limited to absorption of dyes, spectroscopically (e.g., circular dichroism, NMR), size exclusion chromatography, ultracentrifugation, specific DNA binding (e.g., at physiological temperatures as opposed to lower temperatures), and specific protein binding (e.g., SV40 large T antigen only binds to the wild-type active conformation and not the inactive conformation).

As noted above, many of the commonly encountered p53 mutations cannot bind DNA at physiological temperatures, but will bind DNA at lowered temperatures. Therefore, one aspect of measuring conformation of the protein of the p53 family in the presence of test compounds is the temperature dependence. Preferably, conformation is measured at physiological temperatures (around 38°C); an appropriate range is between 20°C and 50°C, and more preferably between 35°C and 42°C. Conformation of the target protein can also be measured over time, from a few minutes to several hours or more. When a wild-type p53 protein or p53 DBD is used in the screen, heating is generally performed longer and at higher temperatures than when a mutant p53 DBD is used. One of skill in the art can easily determine the appropriate temperature using the information provided herein.

Additionally, one can assay both binding of a compound and any change in conformation of a protein of the p53 family simultaneously. In such an assay, a change in conformation of a protein of the p53 family in the presence of a test compound is scored as a hit. Illustrated below by way of non-limiting examples are high-through put screens which assay for compounds that interact with the p53 DBD to cause a conformational change. These high-through put screens were able to identify a class of compounds for use in the methods of the invention. At near-physiologic temperatures, these compounds enhanced the stability of the conformation-sensitive epitope for mAb1620 on wild-type and a variety of mutant p53 proteins. Low micromolar concentrations of compound transiently enhanced the conformational stability of the epitope within living cells and enabled mutant p53 to activate transcription. As described more fully below, an organic non-peptide compound modulated p53 conformation and function when administered to mice harboring tumors with mutant p53 and significantly inhibited the growth of human tumor xenografts with naturally mutated p53.

### c. Cell Based and Animal Based Assays

Once candidate compounds are identified using the primary screen(s) described above, cell-based and animal based assays are generally conducted to determine the effect of the candidate compounds in these systems. Initial assays can involve cell lines derived from tumors having a mutant gene encoding a protein of the p53 family, or cell lines manipulated to express a mutant protein of the p53 family. The effect of the candidate compounds on any one (or all) of the wild-type activities of a protein of the p53 family is assessed. For example, induction of WAF 1 in the presence of the candidate compound indicates that the compound preserves function in mutant p53 by promoting specific DNA binding properties rather than indiscriminate binding properties. Any gene upregulated or down-regulated by p53, or other members of the p53 family, can be examined. Other activities of proteins of the p53 family include growth suppression and apoptosis. Growth suppression is easily assessed in tissue culture cells microscopically or by a colony formation assay. Apoptosis can be visualized by TUNEL staining or propidium iodide staining and flow cytometry.

Additionally, animal-based models can be used to screen for both toxicity and effectiveness of candidate compounds. For example, tumors having mutant p53 can be induced in an animal model, and candidate compounds administered to the animal. Toxicity and tumor growth or regression is assessed. A working example of such a screen is provided below.

### 3. Sources of Compounds For Screening

Compounds that can be screened in accordance with the invention include but are not limited to small organic molecules that are able to gain entry into a cell and affect activity of a protein of the p53 family. A number of compound libraries are commercially available from companies such as Pharmacopeia, Arqule, Enzymed, Sigma, Aldrich, Maybridge, Trega and PanLabs, to name just a few sources. One can also screen libraries of known compounds, including natural products or synthetic chemicals, and biologically active materials, including proteins, for compounds that interact with the p53 DBD. However, preferred compounds are not proteins or peptides (i.e., a string of 3 or more amino acids linked by peptide bonds). Antibodies are peptides that are immunoglobulins or a antigen binding fragments of an immunoglobulin; preferred compounds are also not antibodies. Specific classes and examples of compounds for use in the methods of the invention are described below.

Once a compound that promotes a wild-type activity of a protein of the p53 family is identified, molecular modeling techniques can be used to design variants of the compound that are more effective. Examples of molecular modeling systems are the CHARM (Polygen Corporation, Waltham, MA) and (QUANTA programs Molecular Simulations Inc., San Diego, CA). CHARM performs the energy minimization and molecular dynamics functions. QUANTA performs the construction, graphic modeling and analysis of molecular structure. QUANTA allows interactive construction, modification, visualization, and analysis of the behavior of molecules with each other.

For example, once a compound that a promotes a wild-type activity of a protein of the p53 family is identified, the compound can be used to generate a hypothesis. As will be further detailed below, a preferred hypothesis is that of a planar polycyclic hydrophobic group spaced about 5 (five) to 9 (nine) Angstroms, and more preferably 6 (six) to 8 (eight) Angstroms away from a polar amine. Such a hypothesis can be generated from any one of the compounds of the present invention using the program Catalyst (Molecular Simulations Inc., San Diego, CA). Further, Catalyst can use the hypothesis to search proprietary databases, the Cambridge small molecule database (Cambridge, England), as well as other databases mention *supra*, to identify additional examples of the compounds of the present invention.

Compounds of the present invention can further be used to design more effective variants using modeling packages such as Ludi, Insight II, C²-Minimizer and Affinity (Molecular Simulations Inc., San Diego, CA). A particularly preferred modeling package is MacroModel (Columbia University, NY,NY).

The compounds of the present invention can further be used as the basis for developing a rational combinatorial library. Such a library can also be screened for more effective compounds. While the nature of the combinatorial library is dependent on factors such as the particular compound chosen from the preferred compounds of the present invention to form the basis of the library, and the desire to synthesize the library using a resin, it will be recognized that the compounds of the present invention provide requisite data suitable for combinatorial design programs such as C²-QSAR (Molecular Simulations Inc., San Diego, CA).

The invention having been described, the following examples are offered by way of illustration and not limitation.

### VI. Example 1: p53 DBD Thermostabilization Assay

A high through-put assay using wild-type p53 DBD was developed. Pharmacological compounds were screened using the assay, and those compounds that stabilized the active conformation of the DBD were scored as hits.

### A. Materials and Methods

**Thermostabilization Assay.** Recombinant DBD (residues 94-312) from wild-type and mutant p53 proteins and FLAG-tagged p53 DBD were prepared as described (Pavletich *et al*., 1993, Genes and Dev. 7, 2556-2564; Bullock *et al*., 1997, *supra*.). Mutant proteins used were 143A, 173A, 175S. 249S, and 273H. A number of small molecule organic compounds were tested. Compound stocks were dissolved in DMSO at 10 mg/ml and diluted prior to use. The proteins (0.25-1.0 ng/well) were diluted in a buffer containing 25 mM HEPES, pH 6.8, 150 mM KCl, 10 mM dithiothreitol and attached in 50 ul to Reacti-Bind microtiter plates (Pierce) for 35 minutes on ice. The wells were rinsed with 25 mM HEPES, pH 6.8, 150 mM KCl, compound or diluted DMSO vehicle added, and the plates incubated at the indicated temperatures. Incubation was terminated by placing the wells on ice; ELISA assays performed while maintaining the plates on ice in order to avoid further alterations of the epitopes. Wells were blocked for 1 hour with cold 5 percent skim milk (Difco) in HEPES/KCl buffer prior to addition of the primary antibodies. Monoclonal antibodies mAb1620, mAb240 (Calbiochem) and anti-FLAG M2 antibody (Eastman Kodak Company) were diluted at 1:100-1:250 in HEPES/KCl and added at 100 ul/well for 30 minutes. The plates were rinsed twice with cold HEPES/KCl buffer and incubated with horseradish peroxidase (HRP)-conjugated anti-mouse IgG (Boehringer Mannheim) for another 30 minutes. The HRP signal was developed using TMB developer (Pierce) and the optical density of the signal was read on a BioRad microplate reader set at 450 nm.

### B. Results

**Conformation of p53 DBD is thermolabile.** The epitope recognized by mAb1620 is conformation dependent and its presence on p53 is tightly associated with the protein's tumor suppressor activity (Ball *et al*., 1984, *supra*; Gamble and Milner, 1988, *supra*). Conversely, the epitope recognized by mAb240 is a linear epitope which is exposed when p53 is inactivated by mutation or when wild-type p53 is denatured (Bartek *et al*., 1990, Oncogene 5, 893-899; Stephen and Lane, 1992, J. Mol. Biol. 225, 577-583). Recombinant human p53 DBD (residues 94-312) underwent a transition from the active to the inactive conformation *in vitro,* gradually losing the 1620 epitope while accumulating the 240 epitope. Purified p53 DBD that was immobilized on microtiter plates was heated to near physiologic temperatures and probed with mAb1620 in an ELISA format. The 1620 epitope was lost in a temperature and time dependent manner (Figure 1A). Loss of the 1620 epitope was specifically related to loss of conformation, since a FLAG epitope that was attached to the DBD remained fully stable (Figure 1B). Furthermore, loss of the 1620 epitope occurred in concert with the enhanced appearance of the 240 epitope, assuring that loss of the 1620 epitope reflected a conformational change in the p53 DBD and not loss of the immobilized protein.

The half-life of the 1620 epitope on wild type p53 DBD was approximately 35 minutes at 23°C and decreased progressively at higher temperatures to less than 5 minutes at 45°C (Figure 1A). In parallel, the DNA binding capacity of p53 DBD in gel shift assays was reduced upon heating in solution (data not shown). The half life the 1620 epitope on wild-type p53 DBD was approximately twice that of the position 143 mutant DBD at 37 C (Figure 1C). This finding is consistent with previous reports of reduced thermodynamic stability for several other mutant p53 proteins and establishes that the 1620 epitope may be utilized to monitor the conformation of p53 DBD (Bullock *et al*., 1997, *supra*).

**Compounds stabilize p53 conformation.** The ELISA assay was used to identify compounds that stabilize the active p53 conformation and allow mutant proteins to better retain wild-type functions. Several compounds suppressed the loss of the epitope for mAb1620 at physiologic temperature (for examples see Figure 2A). The relative potency of the compounds was established in titration experiments by determining the concentration required to stabilize 50% of the epitope for mAb1620. Active compounds stabilized the epitope in a dose dependent manner (Figure 2B). The DMSO solvent and several analogues of the active compounds failed to stabilize (Figure 2B, see Tables 1 and 2). Full length wild-type p53 was also stabilized by compounds as were the DBD from several mutant p53 proteins (Data not shown, Figure 2C). In the presence of compound, the mutant proteins were as stable as the wild-type protein in the absence of compound.

While the compounds preserved the epitope for mAb1620, they did not rescue p53 that had already lost the epitope. For example, there was no increase in mAb1620 reactivity when p53 DBD was heated prior to addition of Compound Y. Although the rate of epitope loss was reduced with the compound present, prolonged heating resulted in eventual loss of the 1620-positive conformation. Furthermore, the compound did not appear to be irreversibly bound to p53 since the addition and wash-out of Compound Y prior to incubation at 37°C did not prevent loss of the epitope (data not shown). These findings are consistent with a model where the interaction of p53 DBD with compound enables the protein to more stably retain the functional conformation as recognized by mAb1620.

**Structure of the active compounds.** All of the active compounds identified join together a hydrophobic group (planar polycyclic) and a cationic group (often an amine) by a linker of a specific length. Benzimidazole, benzoquinoline, phenothiazine, and styrylquinazoline in the hydrophobic (R1) position were active whereas subtle changes in these groups and simple bicyclic or monocylic groups were not active under the particular conditions tested (Table 1). Compounds were termed "active" in this assay if there was a greater than 10 fold difference between two matched pairs (see Table 1) of the amount of compound needed to stabilize 50% of the epitope for mAb1620. Thus, it should be noted that compounds termed inactive according to this assay were not absolutely inactive, only relatively inactive. Accordingly, active cationic (R2) groups included dimethylamine, diethyl amine, diethanol amine, methyl amine, methyl piperazine, and morpholine (Table 1). Certain larger amines were correspondingly more active when tested in the phenothiazine series. Negatively charged or uncharged groups such as carboxyl or benzene groups in the R2 position were inactive as defined in this assay (Table 1). The spacing between the R1 and R2 groups was also important for compound activity in this assay as linkers shorter than a propyl length reduced relative compound activity (Table 2). Butyl linkers were slightly less potent than propyl linkers, whereas longer linkers were observed in compounds that exhibited less activity in this assay (Table 2 and data not shown). Branched linkers which retain the correct distance were generally more active than the corresponding linear linkers. These general observations do not limit the scope of the invention but can be used in the practice of the invention to design further molecules.

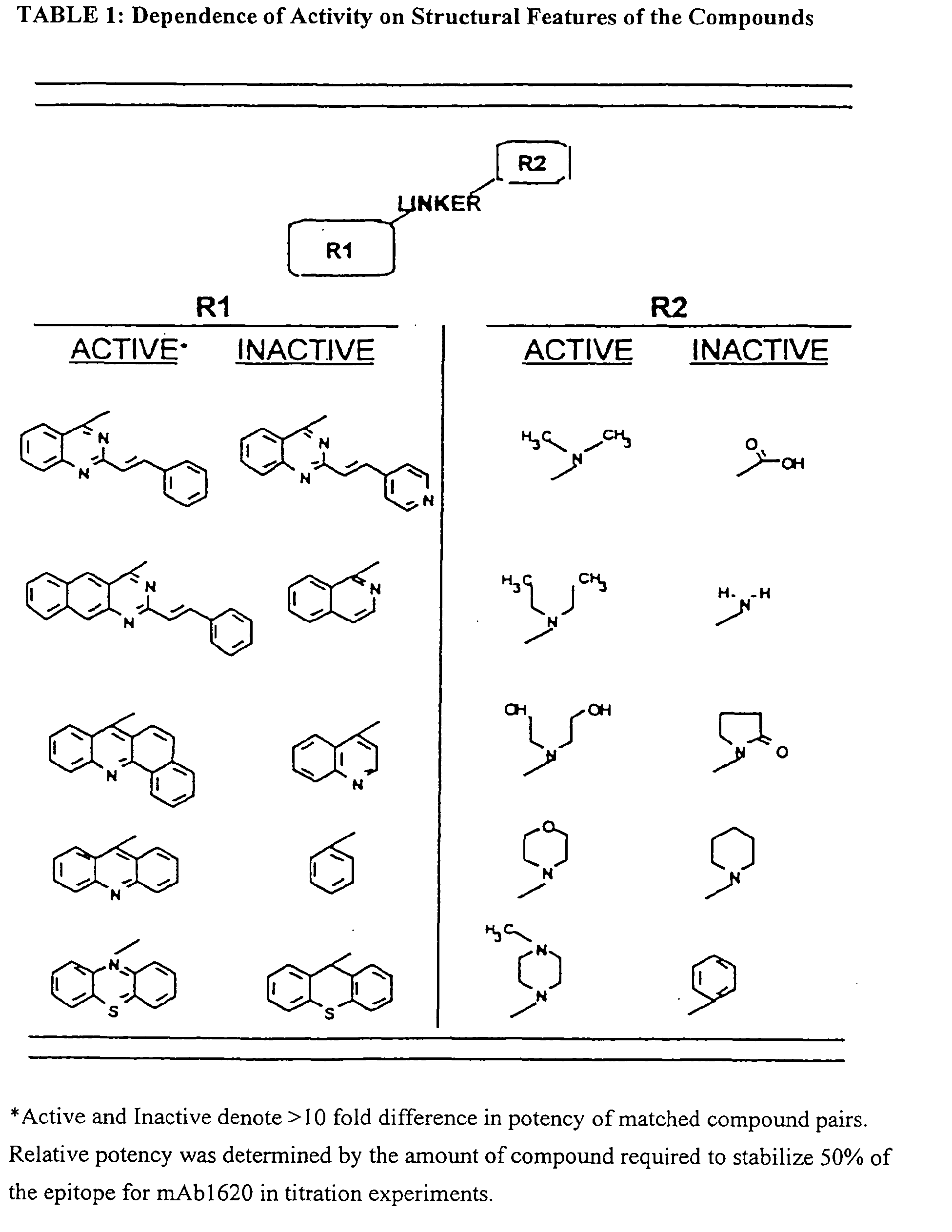

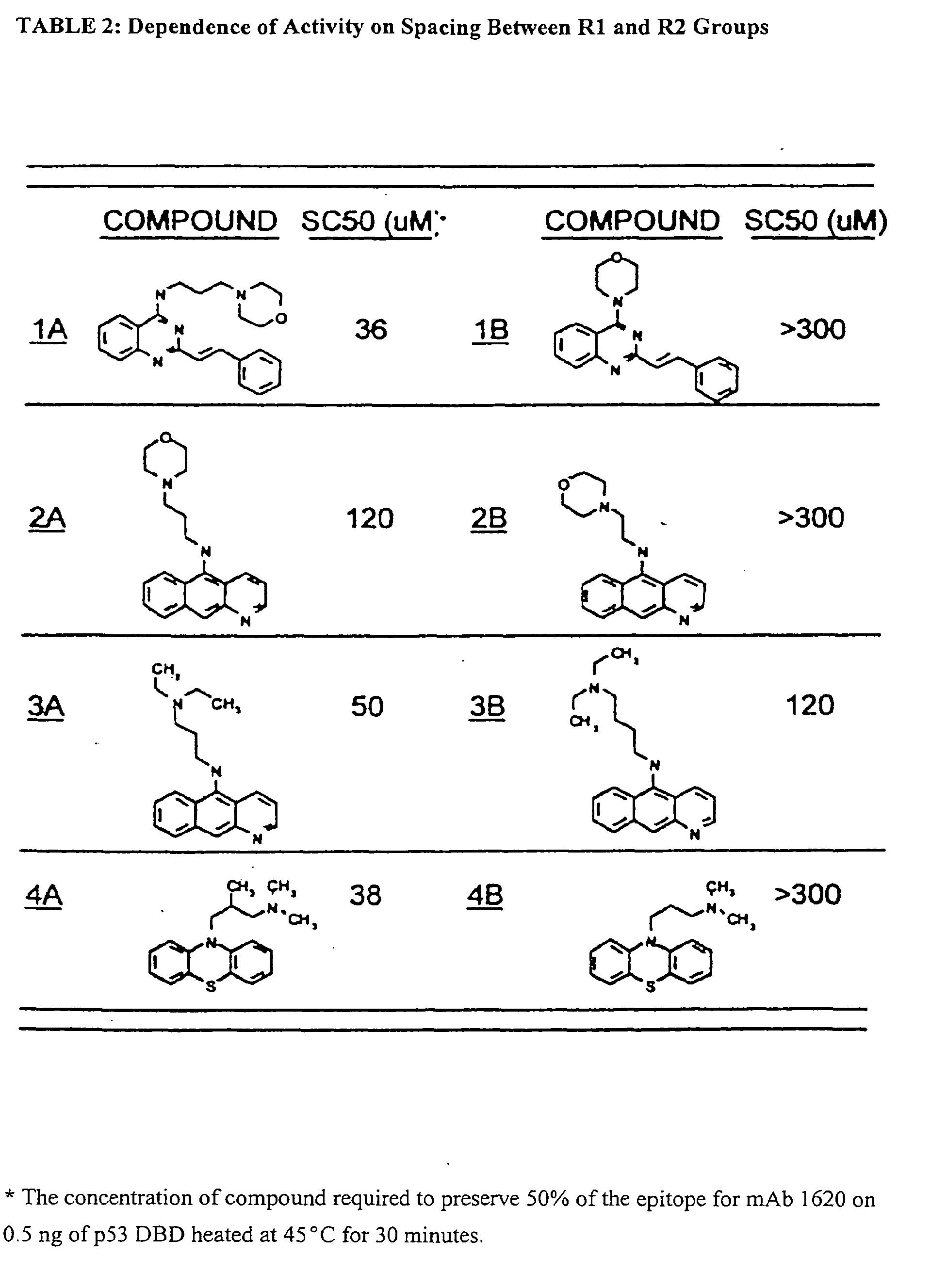

### C. Discussion

The results demonstrate proof of principle for a novel strategy for restoration of mutant p53 function and the development of anticancer therapeutics. This example reports the discovery of the first family of compounds able to act on the isolated DBD to promote its conformational stability.

### VII. Example 2: Determination of p53 Conformation in Cells and Tumors

In this example and the examples that follow, prototype compounds are shown to function at low micromolar concentrations to modulate mutant p53 in living cells and in tumors and to suppress the growth of tumors with naturally mutated p53.

### A. Materials and Methods

**Cell Culture.** All cell lines were obtained from the ATCC and grown in the recommended media with 10 percent fetal calf serum (Gibco BRL).

**Determination of p53 Conformation** Approximately 1x 10⁷H1299/ Reporter + Mutant p53 cells were treated overnight, rinsed three times with cold Tris buffered saline, and lysed in 1.5 ml of hypotonic lysis buffer (20 mM HEPES, pH 7.4, 10 mM NaCl, 20 percent glycerol, 0.2 mM EDTA, 0.1 percent Triton-X 100, 10 mM dithiothreitol with protease inhibitors). Cells were pelleted in microfuge tubes at 2000 rpm for 5 minutes at 4°C and nuclear extracts were prepared by resuspending the pellets in the same buffer with 0.5M NaCl. Tumors samples were homogenized in a Dounce homogenizer using three volumes of the above buffer with 0.5M NaCl. The lysates were cleared by centrifugation at 10,000 rpm for 10 minutes at 4°C. Nuclear extracts were normalized for p53 content as quantitated from Western blots with mAbDO-1 antibody and p53 was captured onto wells of MaxiSorp F96 plates (Nunc) which had been coated overnight at 4°C with mAbDO-1 at 1 ug/ml in 0.05M carbonate buffer, pH 9.6. The wells were washed with cold PBS, blocked for three hours at 4°C using 4 percent skim milk in PBS, and probed using HRP- conjugated mAb1620 antibody in skim milk. The antibody incubation was for one hour on ice, after which wells were washed three times in PBS with 0.05 percent Tween 20, and TMB substrate was used to develop the signal. A standard curve was established using lysate from temperature shifted (32°C) H1299/ Reporter + Mutant p53 cells which expresssed large quantities of 1620-positive p53. Quantitation of the samples was within the linear range of the standard curve, and was corrected for total p53 in each sample as well as for 1620-positive p53 fraction in untreated lysates.

### B. Results

**Stabilization of conformation in cells.** The ability of the compounds to stabilize the 1620-positive conformation of cellular p53 was tested using living cells that express mutant p53 exclusively. H1299 cells, which are null for p53, were transfected with a tumor-derived mutant p53 (position 173) and a non-conformation-sensitive p53 antibody (mAbDO-1) was used in Western blots to select a clone expressing abundant quantities of the mutant protein. Low steady state levels of p53 that displayed the epitope for mAb1620 were detected in extracts from the transfectant, confirming that a small fraction of mutant p53 can retain the active conformation (Chen *et al*., 1993, Oncogene 8, 2159-2166). Low micromolar concentrations of Compound X increased the steady state fraction of 1620-positive p53 in cells by approximately 5-fold (Figure 3A). Maximal levels of epitope enrichment were reached at 4 to 6 hours after treatment. Total amount of p53 was unchanged as measured by reactivity with mAbDO-1 that is directed against a non-conformation sensitive epitope located in the amino terminus of the protein.

### C. Discussion

The results show that conformation-stabilizing compounds identified by the methods of the invention can stabilize the active conformation of p53 in living cells. Compounds that restore mutant p53 in tumors can target either the total non-functional p53 pools or the subset of p53 that displays the epitope for mAb1620. The key target for the compounds described here appears to be newly synthesized mutant p53 that still retains the active conformation. Indeed, compounds enhanced the persistence of the 1620 epitope, but were unable to restore the 1620 epitope that has been lost due to prior heating *in vitro*. Compounds that enhance the stability of the active conformation on newly synthesized p53 would allow the accumulation of steady state levels of functional p53 in a time-dependent manner. The observed four hour delay for achieving maximal 1620 epitope enhancement in cells is consistent with this hypothesis (Figure 3A).

### VIII. Example 3: Restoration of p53 Function

### A. Materials and Methods

**Transactivation assays.** Cells were transfected with expression plasmids encoding mutant p53 proteins (173A, 249S) and a neomycin selectable marker using DOTAP cationic lipid transfection-reagent (Boehringer Mannheim) or calcium phosphate. Cells were also transfected with a plasmid encoding the hygromycin resistance marker and a p53 reporter gene comprised of four copies of a p53 binding sequence corresponding to a p53 binding sequence in the promoter region of the Herpes Simplex virus thymidine kinase gene (base numbers 26 to 58 of GenBank accession no. S57428 thymidine kinase, which begins with the sequence GCCTTGCCT and ends with the sequence TGCCTTTTC) placed upstream of the SV40 basal promoter driving the luciferase gene. A matched cell pair was prepared by transfecting a clone of cells with the reporter construct with an additional construct for mutant p53 expression. Transfected clones were selected for growth in media containing Hygromycin or G418, as appropriate. Monolayers of cells in 96-well tissue culture plates (Costar) were treated with compound, and luciferase activity was determined using a substrate conversion assay (Promega) and quantitated with a Dynatech microplate luminometer.

**WAF1 and p53 Expression.** Cultured cells were treated for 21 hours, rinsed 3 times with cold Tris buffered saline, scraped, and pelleted at 10,000 rpm for 30 seconds before resuspending them in 50 mM HEPES, pH 7.5, 0.1 percent NP-40, 250 mM NaCl, 5 mM EDTA, 50 mM NaF, 1 mM DTT, 50 ug/ml aprotinin, 1 mg/ml Pefabloc (Boehringer Mannheim). Protein concentrations were determined using Bradford reagent (BioRad) and 5 or 10 ug of cell lysate were loaded onto 8-16 percent gradient polyacrylamide/SDS gels (Novex). Proteins were transferred onto Immobilon P membrane ( Millipore) in Towbin's buffer (Towbin *et al*., 1979, Proc. Nat. Acad. Sci.:USA 76, 4350) with 20 percent methanol. Membranes were bisected between the 32.5 and 47.5 kDa molecular weight markers and blocked for 1 hour at room temperature in SuperBlock (Pierce) plus 3 percent skim milk. The bottom half of the blot was probed for WAF 1 expression using monoclonal antibody clone EA10 (Calbiochem WAF1 Ab-1) and the top half of the blot was probed for total p53 expression using mAbDO-1 (Calbiochem p53 Ab-6). The blots were washed for one hour in three changes of Tris buffered saline with 0.1 percent Tween 20, before the addition of the secondary antibody, HRP-conjugated anti-mouse IgG. The bands were visualized using Renaissance ECL (DuPont) and exposure to Hyperfilm ECL (Amersham Life Science).

### B. Results

**Restoration of p53 function in cells.** To determine if the stabilization of p53 conformation could result in better retention of wild-type functions, we examined the sequence-specific transcription activity of p53. H1299 cells were transfected with a p53-inducible luciferase reporter gene and a stable clone (H1299/Reporter) was secondarily transfected with mutant p53 to obtain a matching clone that expressed both the reporter gene and the position 173 mutant p53 (H1299/Reporter + Mutant p53). Compounds enhanced the transcription activity of the mutant p53 as measured by reporter gene induction (Figure 3B). Low levels of transcription activation were observed in H1299/Reporter cells which may be due to the presence of a p53 homologue, p73 (data not shown). Although we have not yet established whether these compounds can enhance p73 activity, the extensive p53-dependent increase in reporter gene induction suggests that p53 is the primary target in these cells. The p53-dependent activation of the reporter gene occurred within a relatively small concentration range as the effectiveness of the compounds at higher doses was limited by cell detachment. Enhancement of transcription activity peaked at 12-16 hours after treatment (data not shown). This observation is consistent with reporter gene expression occurring as a secondary event after stabilization of the functional p53 conformation, which occurred at 4-6 hours after treatment.

Compound Y was superior to Compound X in reporter gene induction assays. This may be attributed to a secondary effect of Compound Y involving DNA damage and leading to elevated levels of p53 protein (Figure 3B). Compound Y, but not Compound X, enhanced the total p53 protein levels at concentrations required for cellular activity. To ensure that DNA damage is not solely responsible for p53 reporter gene induction by Compound Y, we tested the effects of the DNA damaging agent Adriamycin. Adriamycin did not induce the reporter gene within a wide range of concentrations (0.4 to 40 ug/ml) despite its ability to induce mutant p53 accumulation in cells (data not shown). These results demonstrate that conformational stabilization, but not the accumulation of mutant p53, can promote specific transcription activity. In particular, Compound X, which does not elevate the steady state levels of total p53 protein, appears to restore p53 transcription function uniquely through conformational stabilization.

Compound X up-regulated WAF1, a p53-responsive cellular gene product, in the presence of mutant p53. Saos-2 osteosarcoma cells, which do not express p53, were transfected with mutant p53 expression vectors and clones expressing either of two mutants (position 173 or position 249) were isolated. The clones expressed lower basal levels of WAF1 as compared to the parental Saos-2 cells, possibly reflecting our selection of faster growing clones. These cells were treated with Compound X for 16 hours and lysates representing equal amounts of protein were analyzed on Western blots for p53 and WAF1. Cells which expressed either of the two mutant p53 proteins, but not the parental Saos-2 cells, had elevated expression levels of WAF 1 upon treatment (Figure 4). The total amount of p53 protein in these lysates was essentially unchanged. Adriamycin did not induce WAF-1 expression in Saos-2 cells with mutant p53, although it did elevated WAF-1 expression in U2OS cells which express wild-type p53 (data not shown).

### C. Discussion

The mode of action of the conformation-stabilizing agents described here is clearly distinct from that observed for traditional cytotoxic anti-neoplastic agents. Cytotoxic agents that are used in cancer chemotherapy are generally ineffective in cells with mutant p53 (Lowe *et al*., 1993, Nature 362, 847-849; O'Connor *et al*., 1997, Cancer Res. 57, 4285-4300). In fact, the DNA damaging agent, Adriamycin, did not restore mutant p53 for transcription activity in our assays. Cytotoxic compounds are also hallmarked by pronounced induction of total p53 protein in normal and tumor cells. Compound X did not induce the total p53 protein levels in cells or in tumors. As p53 induction is a sensitive measure of cellular DNA damage, it is unlikely that Compound X can damage DNA at efficacious concentrations. Taken together, our findings indicate that the stabilization of the 1620 positive conformation and functional restoration of mutant p53 activity can occur via a DNA damage-independent mechanism.

Several lines of evidence preclude a non-specific effect on protein stabilization. Glycerol, a non-specific inhibitor of protein denaturation which functions by displacing water and creating a more hydrophobic microenvironment around protein molecules, can restore the nuclear localization of a mutant mouse p53 in cells at a concentration of 600 mM (Brown *et al*., 1997, J. Clin. Invest. 99, 1432-1444). Compound X was active at 0.03 mM in this assay, suggesting a much more precise interaction involving specific contacts between the compound and p53 (data not shown). Furthermore, the observation that Compound X can affect p53 conformation in the presence of a vast excess of other proteins in culture and *in vivo* (see below) is consistent with selective recognition of p53. Still, the nature of compound interaction with p53 may not involve tight binding to the native protein structure. A strong interaction with a small subset of the protein molecules that are in a transition state may function to block further deviation from the active conformation or facilitate reversion to the native conformation.

### IX. Example 4: Tumor Growth Assay

### A. Materials and Methods

**Tumor growth assay**. Cultured cells were rinsed with PBS and 1 X 10⁶ A375.S2 or 5 X 10⁶ DLD 1 cells inoculated in 90 percent Matrigel (Becton Dickinson) unilaterally into the right flanks of 20 gram female NU/NU-nuBR mice (Charles River Laboratories). Compound X was administered intraperitoneally in a saline solution with in 0.1% Pluronic P-105 (BASF). Tumor diameter was measured in two dimensions using calipers, and converted to tumor volume (Euhus *et al*., 1986, J. Surg. Oncol. 31, 229-234).

### B. Results

**Modulation of p53 in *vivo.*** Compound X enhanced the steady state levels of p53 fraction that displays the epitope for mAb1620 in tumors with mutated p53. Compound was administered intraperitoneally at 100 mg/Kg to mice bearing subcutaneous tumors derived from injected H1299/Reporter + Mutant p53 cells. Animals were sacrificed after a single dose of the compound and tumor lysates were analyzed for total and 1620-positive p53 expression. Total p53 levels were unchanged as measured on Western blots with mAbDO-1. The lysates were normalized for the minor variations in total p53 content and tested in an ELISA assay for expression of the epitope for mAb1620. The epitope was increased within 3 to 5 hours after treatment (Fig. 5). The time course of the response *in vivo* was similar to that of the cultured cells (Figure 3A).

In order to evaluate the functional restoration of mutant p53 *in vivo*, we assessed the expression of the luciferase reporter gene in tumors from treated and untreated animals. A maximum 4.5-fold induction of the reporter gene was observed at 8 hours after dosing (Fig 5). The time lag between the conformational and the functional responses may reflect the time required for translation of the luciferase transcript and accumulation of the protein. The peak plasma concentration of compound in mice was approximately 10 ug/ ml, which is below what would be required for maximal induction of the reporter gene in cells (data not shown). Therefore, the lower levels of reporter gene induction in tumors as compared to the cultured cells may be due to suboptimal exposure.

### C. Discussion

The results show that conformation-stabilizing compounds can functionally restore a number of randomly chosen mutants. Thus, the methods and compounds of the invention are broadly applicable to different p53 mutants. For example, the position 241 mutation in DLD-1 cells, which affects a minor DNA contact site, can be functionally complemented through the stabilizing activity of Compound X. Therefore, a great many of the p53 mutants, including some at the DNA contact sites, can be restored upon stabilization of the active conformation.

Compound X demonstrated therapeutic selectivity *in vivo* despite stabilizing the conformation of both wild-type and mutant p53 *in vitro.* Indeed, the compound appeared safe and no mortality was observed when mice were dosed at 200 mg/kg/day (100 mg/kg b.i.d.) for 14 consecutive days (data not shown). The selectivity may be due to the very low steady state levels of p53 in normal cells as compared to much higher levels in tumor cells (Lassus *et al*., 1996, EMBO J. 15, 4566-4573). Also, tumor-specific stresses such as DNA lesions and oxygen or nutrient deprivation may preferentially prime tumor cells for the apoptotic effects of p53 (Chen *et al*., 1996, Genes and Dev. 10, 2438-2451). If so, it may be possible to achieve synergistic anti tumor effects by combining p53 stabilizing compounds with radiation or genotoxic therapeutics.

The foregoing written specification is sufficient to enable one skilled in the art to practice the invention. Indeed, various modifications of the above-described means for carrying out the invention which are obvious to those skilled in the field of molecular biology, medicine or related fields are intended to be within the scope of the following claims.

## Claims

1. A method of screening an organic non-peptide compound for interaction with one or more domains of either a wild-type human protein of the p53 family or a mutant human protein of the p53 family, which interaction can stabilize a functional conformation thereof under physiological conditions and promote wild-type activity of the protein of the p53 family, or restore wild-type activity to a mutant protein of the p53 family, which method comprises contacting said human protein of the p53 family, or one or more domains thereof, and said organic non-peptide compound, and monitoring the interaction thereof.

2. A method as claimed in claim 1, wherein the protein of the p53 family is wild-type.

3. A method as claimed in claim 1, wherein the protein of the p53 family is a mutant encoded by an allelic variant.

4. A method as claimed in any of claims 1 to 3, wherein said protein is p53, p63 or p73.

5. A method as claimed in claim 4, wherein said protein is p53.

6. A method as claimed in any preceding claim, wherein said protein of the p53 family employed in said screening comprises the full length of the protein of the p53 family.

7. A method as claimed in any of claims 1 to 5, wherein said protein of the p53 family employed in said screening comprises, or contains only, the DNA binding domain of the protein of the p53 family.

8. A method as claimed in claim 7, wherein a fusion protein containing the DNA binding domain of the protein of the p53 family is employed.

9. A method as claimed in claim 7, wherein said protein of the p53 family employed in said screening comprises the DNA binding domain of the protein of the p53 family, together with the N-terminal and / or C-terminal domain of said protein of the p53 family.

10. A method as claimed in any preceding claim, which comprises preparing a reaction mixture of said protein of the p53 family and said organic non-peptide compound so as to allow interaction thereof so as to form a complex comprising said protein of the p53 family and said organic non-peptide compound, which complex is removed and / or detected in said reaction mixture.

11. A method as claimed in any preceding claim, which further comprises, either simultaneously or successively, assaying said organic non-peptide compound for its effect on the function of said protein of the p53 family.

12. A method as claimed in claim 11, which comprises, either simultaneously or successively, assaying said organic non-peptide compound for its effect on the conformation of said protein of the p53 family.

13. A method as claimed in any preceding claim, which further comprises successively assaying said organic non-peptide compound for its effect on one or more wild-type activities associated with a protein of the p53 family, of cells or cell lines derived from tumours having a mutant gene encoding a protein of the p53 family or cells or cell lines derived from tumours manipulated to express a mutant protein of the p53 family.

14. A method of identifying organic non-peptide compounds for interaction with one or more domains of either a wild-type human protein of the p53 family or a mutant human protein of the p53 family, which interaction can stabilize a functional conformation therein and promote the wild-type activity of the protein of the p53 family or restore wild-type activity to a mutant human protein of the p53 family, which method comprises employing molecular modelling to provide a variant of a compound identified by a method as claimed in any of claims 1 to 13.

15. A method of screening an organic non-peptide compound identified in a method as claimed in claim 14 for interaction with one or more domains of either a wild-type human protein of the p53 family or a mutant human protein of the p53 family, which interaction can stabilize a functional conformation therein and promote the wild-type activity of the protein of the p53 family, or restore wild-type activity to a mutant human protein of the p53 family, which method comprises contacting said human protein of the p53 family, or one or more domains thereof, and said organic non-peptide compound, and monitoring the interaction thereof.

16. A method as claimed in claim 14 or 15, wherein said human protein of the p53 family or domain thereof employed is as defined in any of claims 2 to 9.

17. A method as claimed in claim 15 or 16, which comprises preparing a reaction mixture of said protein of the p53 family and said organic non-peptide compound so as to allow interaction thereof so as to form a complex comprising said protein of the p53 family and said organic non-peptide compound, which complex is removed and/or detected in said reaction mixture.

18. A method as claimed in any of claims 15 to 17, which further comprises, either simultaneously or successively, assaying said organic non-peptide compound for its effect on the function of said protein of the p53 family.

19. A method as claimed in claim 18, which comprises, either simultaneously or successively, assaying said organic non-peptide compound for it effect on the conformation of said protein of the p53 family.

20. A method as claimed in any of claims 15 to 19, which further comprises successively assaying said organic non-peptide compound for its effect on one or more wild-type activities associated with a protein of the p53 family, of cells or cell lines derived from tumours having a mutant gene encoding a protein of the p53 family or cells or cell lines derived from tumours manipulated to express a mutant protein of the p53 family.
